(19) Europäisches Patentamt
European Patent Office
Office européen des brevets

(11) **EP 4 645 340 A1**

(12) **EUROPEAN PATENT APPLICATION**
published in accordance with Art. 153(4) EPC

(43) Date of publication:
05.11.2025 Bulletin 2025/45

(21) Application number: 23909367.7

(22) Date of filing: 19.09.2023

(51) International Patent Classification (IPC):
*G16H 50/70* (2018.01)

(52) Cooperative Patent Classification (CPC):
G06F 17/18; G16H 10/60; G16H 50/70

(86) International application number:
PCT/CN2023/119663

(87) International publication number:
WO 2024/139432 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 26.12.2022 CN 202211676345

(71) Applicants:
• Peking University Third Hospital (The Third
Clinical Medical School of Peking University)
Beijing 100191 (CN)
• Hangzhou Qingguo Medical Technology Co., Ltd.
Hangzhou, Zhejiang 310000 (CN)

(72) Inventors:
• XU, Huiyu
Beijing 100191 (CN)
• LI, Rong
Beijing 100191 (CN)
• QIAO, Jie
Beijing 100191 (CN)
• FENG, Guoshuang
Beijing 100191 (CN)
• HAN, Yong
Hangzhou, Zhejiang 310000 (CN)

(74) Representative: Keller Schneider
Patentanwaltsgesellschaft mbH
Linprunstraße 10
80335 München (DE)

(54) **METHOD AND SYSTEM FOR CONVERTING DETECTED HORMONE DATA**

(57) A method for converting hormone data, which is detected by means of different detection methods. The method comprises: acquiring a first detection value for a hormone, which first detection value is from a first platform; acquiring a second detection value for the hormone, which second detection value is from a second platform; fitting the first detection value and the second detection value by using Passing-Bablok regression; determining, on the basis of the Passing-Bablok regression fitting result, whether there is a systematic difference between the first detection value and the second detection value; if there is no systematic difference between the first detection value and the second detection value, converting the first detection value or the second detection value on the basis of a fitting formula of Passing-Bablok regression; and if there is a systematic difference between the first detection value and the second detection value, fitting the first detection value and the second detection value on the basis of spline regression, and converting the first detection value or the second detection value on the basis of a fitting formula of spline regression.

EP 4 645 340 A1

**Description**

**TECHNICAL FIELD**

The present application relates to a method and system for transforming hormone detection data obtained through different detection methods, particularly a method and system for transforming sex hormone detection data.

**BACKGROUND**

Hormones are substances that are secreted directly into the blood by the endocrine organs or tissues of humans and animals and have special effects on the body. Digestive tract organs and tissues like the placenta also secrete hormones, such as secretin, gastrin, and chorionic gonadotropin, etc. The coordinated action of various hormones is essential for maintaining the body's metabolism and function. In terms of chemical properties, some hormones are phenolic derivatives, such as adrenaline and thyroxine, etc.; some are polypeptides or proteins, such as pituitary hormone-releasing factors, pituitary hormones, insulin, glucagon, calcitonin, and parathyroid hormone, etc.; some are steroid compounds, such as androgens, estrogens, and adrenal cortical hormones, etc. Many hormone preparations and synthetic products have important uses in both medicine and animal husbandry.

An important category of hormones is sex hormones. Sex hormones (chemical nature is lipid) refer to hormones synthesized by the gonads of animals, as well as the placenta, zona reticularis of the adrenal cortex and other tissues. These hormones play a role in promoting the maturation of sexual organs, the development of secondary sexual characteristics, and maintaining sexual functions, etc. Female animals' ovaries primarily secrete two types of sex hormones-estrogen and progesterone, while male animals' testes mainly secrete androgens, primarily testosterone. The hormones secreted by the hypothalamus-pituitary-gonadal axis or the hypothalamus -pituitary-adrenal axis are generally classified into three categories based on their chemical structures. The first type is steroids, such as adrenal cortex hormones and steroidal sex hormones. The second type is amino acids, including thyroid hormone, adrenal medullary hormone, pineal hormone, etc. The structures of the third type of hormones are peptides and proteins, such as hypothalamic hormones, pituitary hormones, gastrointestinal hormones, calcitonin, etc. The third type of hormones are basically protein macromolecular substances, and from an immunological perspective, these hormones possess multiple antigenic epitopes and can generally be detected using the double antibody sandwich method.

Anti-Miillerian hormone (AMH) assists in the regression of the Mullerian ducts during male embryonic sex differentiation, first discovered by Jost in 1947. In 1999, Dulinger et al. used AMH gene knockout mice to discover that AMH is responsible for inhibiting the recruitment of primordial ovarian follicles, and AMH belongs to the third type of hormones mentioned above. Since then, researchers have begun research and clinical applications of AMH in reproductive science and developed a kit for detecting AMH. During ovarian stimulation, AMH is an important marker for assessing ovarian reserve. AMH is also considered an ideal marker for individualized ovarian stimulation protocols that determine the dose of human recombinant follicle-stimulating hormone (rFSH) promoting adrenal cortex hormones in IVF treatment regimens. With AMH being increasingly recognized as a crucial parameter in reproductive science, precise, efficient, and robust AMH detection methods have become particularly critical.

Currently, various methods for AMH detection exist in the relevant field, with the new automated anti-Müllerian hormone detection method gradually replacing the original ELISA method. Currently in the market, commercialized automated anti-Müllerian hormone (AMH) testing methods include the Elecsys AMH method provided by Roche (hereinafter referred to as the "Roche method" or "Elecsys method"), the Access AMH method provided by Beckman Coulter (hereinafter referred to as the "Beckman method" or the "Access method"), the chemiluminescent AMH detection method provided by Kangrun (hereinafter referred to as the "Kangrun chemiluminescent method"), and the enzyme-linked immunosorbent assay (ELISA) AMH detection method provided by Kangrun (hereinafter referred to as the Kangrun Anshlab method or the Kangrun ELISA Anshlab method).

Roche launched the Elecsys method in 2014, which is a test based on the monoclonal antibodies F2B 12/H and F2B 7/A. Subsequently, Beckman Corporation also launched the Access method using the same monoclonal antibody. Since Roche and Beckman use similar antibodies, their main distinction lies in the development of their detection methods. In addition, China's Kangrun Company has developed an automated AMH detection method using a different monoclonal antibody, the code for which is 1B63D7.

Estrogen is a main class of female hormones. It promotes the maturation of female secondary sexual organs and the development of secondary sexual characteristics, maintaining normal sexual desire and reproductive functions, primarily in the form of estradiol ($E_2$). It is inactivated in the liver, converted into estriol and estrone, and excreted in the urine after combining with glucuronic acid. During pregnancy, the placenta can secrete a large amount of estriol. Estradiol ($E_2$) was first discovered 77 years ago by Edward Doisy and has been shown to play a key role in a variety of physiological processes. Therefore, quantification of $E_2$ has been widely used in clinical assessment of female or male conditions or diseases, such as fertility testing, monitoring of ovarian stimulation during assisted reproductive technology, and detection of pregnancy, polycystic ovary syndrome (PCOS), hypogonadism, amenorrhea, and $E_2$-related tumors, etc. Methods used for $E_2$ quantification include immunoassay, gas chromatography-mass spectrometry (GC-MS), and liquid chromatography-tandem mass spectrometry (LC-MS/MS). Mass spectrometry methods have better specificity and sensitivity than immunoassays, but their applications as routine clinical methods are limited due to cost and technical complexity. Automated immunoassay platforms that do not require complicated sample extraction processes have become the preferred choice for most clinical laboratories due to their high throughput, reproducibility, and shorter turnaround time.

Most direct automated immunoassay platforms are optimized to measure $E_2$ concentrations between 20 and 2000 pg/mL. Such a concentration range can almost meet all clinical purposes in obstetrics and gynecology, and can also meet the needs of assisted reproductive technology (ART) in hospitals. However, it is not easy to standardize $E_2$ measurements over such a wide concentration range. It has been reported that the determination sensitivity of low-level $E_2$ check is affected. In the hospital clinical laboratory, clinicians found that there was great heterogeneity between different automated device immunoassay platforms, especially when $E_2$ levels were low, such as <200pg/ml. This may be due to the lack of an international calibrator for $E_2$, differences in calibration point assignments, and variations in detection systems. Changes in $E_2$ levels, especially within low range, are bound to cause great confusion among clinicians in interpreting the results.

**SUMMARY**

Given that the several major sex hormones, such as AMH and $E_2$, as described in the above background technology, all play significant roles in clinical detection and diagnosis, therefore, the existence of different methods and platforms for detecting these hormones can lead to inconsistencies in evaluation and analysis of detection results from different sources.

Taking AMH as an example, there are currently different AMH detection methods on the market, and the AMH values obtained by these methods are inconsistent. Currently, the AMH results of patients obtained using a certain AMH testing method in a certain hospital cannot be adopted by hospitals using other AMH testing methods, significantly increasing the medical burden on patients and complicating clinical practice, and hindering the clinical application of currently available online assessment tools related to AMH. Using AMH as one of the most important predictive indicators, the inventors of the present application have previously established several online prediction tools, including an ovarian reserve assessment tool (http://121.43.113.123:9999/), a PCOS screening assessment tool (http://121.43.113.123:8888/ ), an oocyte retrieval prediction tool (http://121.43.113.123:8002/), and an online tool for guiding the initiation and adjustment dosages of exogenous FSH (http://121.43.113.123:8004). Given that AMH is an increasingly important evaluation parameter in reproductive medicine, if it is desirable that the AMH data of patients tested by different hospitals and testing institutions can be easily applied and calculated in a unified manner, there is an urgent need for a calculation method and system capable of converting or unifying AMH values obtained from different detection methods.

Taking estradiol as an example, estradiol is a C18 steroid hormone and the most biologically active hormone among estrogens. It is produced by the male testes, female ovaries, and placenta during pregnancy, with its levels varying throughout the menstrual cycle. The primary physiological function of estradiol is to promote the formation and development of ovarian follicles, promote the development of the ovaries and female reproductive organs, promote the emergence of secondary sexual characteristics in women, and also promote the liver's synthesis of various transport proteins. The concentration of circulating blood estradiol is one of the indicators for assessing the function of the hypothalamus-pituitary-reproductive target gland axis, and it has certain diagnostic significance for endocrine diseases and gynecological diseases such as precocious puberty, dysplasia, infertility, etc. In the hospital clinical laboratory, clinicians found that there was great heterogeneity between different automated device immunoassay platforms, especially at low $E_2$ levels, such as <200pg/ml. This is probably due to the lack of an international calibrator for $E_2$ and the different assignment of calibration points. Changes in $E_2$ levels, especially within low range, are bound to cause great confusion among clinicians in interpreting the results.

Specifically, the technical solutions of this application are as follows:

1. A method for converting hormone data detected by different detection methods, comprising:

obtaining a first detection value for the hormone from a first platform;
obtaining a second detection value for the hormone from a second platform;
using Passing-Bablok regression to fit the first and the second detection values;
determining whether there is a systematic difference between the first and the second detection values based on the Passing-Bablok regression fitting results;
if converting the first detection value or the second detection value based on the fitting formula of Passing-Bablok regression, if there is no systematic difference between the first and the second detection values;
fitting the first and the second detection values based on the spline regression, and converting the first detection value or the second detection value based on the fitting formula of spline regression, if there is a systematic difference between the first and the second detection values.

2. The method according to item 1, wherein the determination of whether there is a systematic difference between the first and the second detection values based on the Passing-Bablok regression fitting results is made by calculating the 95% confidence interval of the intercept from the Passing-Bablok regression results to assess the presence of a systematic difference between the first and second detection values.

3. The method according to item 2, wherein:

the 95% confidence interval of the intercept comprises 0, indicating no systematic difference between the first and the second detection values;
the 95% confidence interval of the intercept does not comprise 0, indicating the presence of systematic difference between the first and the second detection values.

4. The method according to any one of items 1 to 3, wherein
when fitting the first and the second detection values based on the spline regression, intrinsic nodes and statistical indicators are utilized to confirm the nodes of the spline regression.

5. The method according to item 4, wherein the statistical indicator is an AIC (Akaike Information Criterion) or a BIC (Bayesian Information Criterion) indicator.

6. The method according to any one of items 1 to 5, wherein the hormone is a sex hormone, preferably the sex hormone is selected from hypothalamic-pituitary-gonadal axis hormone, comprising hypothalamic hormone, pituitary prolactin (PRL), follicle-stimulating hormone (FSH), luteinizing hormone (LH), anti-Miillerian hormone(AMH), inhibin B, estradiol ($E_2$), testosterone (T), dehydroepiandrosterone sulfate ((DHEA-S), estriol ($E_3$), and progesterone (P); preferably anti-Miillerian hormone (AMH) or estradiol ($E_2$).

7. The method according to item 6, wherein the sex hormone is anti-Miillerian hormone (AMH), wherein when the first platform is the Roche AMH detection platform and the second platform is the Kangrun chemiluminescent AMH detection platform, Passing-Bablok regression is used to convert the detection results of the Roche AMH detection platform and the Kangrun chemiluminescent AMH detection platform;

preferably, the formula for converting Kangrun chemiluminescent AMH (X) to Roche AMH (Y) is:

$$\text{Roche AMH} = a1 + b1*\text{Kangrun chemiluminescent AMH;}$$

wherein a1 is any value selected from a range of -0.0035 to 0.0303; b1 is any value selected from a range of 0.85 to 0.8844;
preferably, the formula for converting Roche AMH (X) to Kangrun chemiluminescent AMH (Y) is:

$$\text{Kangrun chemiluminescent AMH} = a2 + b2*\text{Roche AMH;}$$

wherein a2 is any value selected from a range of -0.0356 to 0.00393; b2 is any value selected from a range of 1.1307 to 1.1765.

8. The method according to item 6, wherein the sex hormone is anti-Miillerian hormone (AMH), wherein when the first platform is the Beckman AMH detection platform and the second platform is the Roche AMH detection platform, spline regression is used to convert the detection results of the Beckman AMH detection platform and the Roche AMH detection platform;

preferably, the formula for converting Roche AMH (X) to Beckman AMH (Y) is:

$$\text{Beckman AMH} = a3 + b3*\text{Roche AMH} + c3*g1 \ (\text{Roche AMH-1}) + d3*g2$$

$$(\text{Roche AMH-9}),$$

in the formula, if Roche AMH is $\leq$ 1 ng/mL, then g1 = 0, g2 = 0, and the formula is:

$$\text{Beckman AMH} = a3 + b3*\text{Roche AMH;}$$

if Roche AMH is >1 ng/mL and ≤9 ng/mL, then g1=1, g2=0, and the formula is:

$$\text{Beckman AMH} = a3 + b3*\text{Roche AMH} + c3*(\text{Roche AMH-1});$$

If Roche AMH is >9 ng/mL, then g1=1, g2=1, and the formula is:

Beckman AMH = a3 + b3*Roche AMH + c3*(Roche AMH-1) + d3*(Roche AMH-9)

Wherein a3 is any value selected from a range of -0.28809 to 0.30461; b3 is any value selected from a range of 0.665236 to 1.430824; c3 is any value selected from a range of 0.073606 to 0.900514; d3 is any value selected from a range of -0.70344 to -0.42116;
preferably, the formula for converting Beckman AMH (X) to Roche AMH (Y) is:

Roche AMH = a4 + b4*Beckmann AMH + c4*g1 (Beckmann AMH-0.5) + d4*g2 (Beckmann AMH-1),

in the formula, if the Beckman AMH value is ≤ 0.5 ng/mL, then g1=0, g2=0, and the formula is:

$$\text{Roche AMH} = a4 + b4*\text{Beckmann AMH}$$

if the Beckman AMH value is >0.5 ng/mL and ≤1 ng/mL, then g1=1, g2=0, and the formula is:

$$\text{Roche AMH} = a4 + b4*\text{Beckmann AMH} + c4*(\text{Beckmann AMH - 0.5});$$

if the Beckman AMH value is > 1 ng/mL, then g1 = 1, g2 = 1, and the formula is:

Roche AMH = a4 + b4*Beckmann AMH + c4*(Beckmann AMH-0.5) + d4*(Beckmann AMH-1);

a4 is any value selected from a range of -0.42466 to 0.41822; b4 is any value selected from a range of -0.11202 to 2.17363; c4 is any value selected from a range of -1.96122 to 1.21734; d4 is any value selected from a range of -0.49419 to 0.66553.

9. The method according to item 6, wherein the sex hormone is anti-Miillerian hormone (AMH), wherein the first platform is the Beckman AMH detection platform, and the second platform is the Kangrun chemiluminescent AMH detection platform, and spline regression is used to convert the detection results of the Beckman AMH detection platform and the Kangrun chemiluminescent AMH detection platform;

preferably, the formula for converting Beckman AMH (X) to Kangrun chemiluminescent AMH (Y) is:

Kangrun chemiluminescent AMH = a5+b5*Beckman AMH+c5*g1(Beckman AMH-1)+d5*g2(Beckman AMH-4)+e5*g3(Beckman AMH-13)

in the formula, if the Beckman AMH value is ≤ 1 ng/mL, then g1=0, g2=0, g3=0, and the formula is:

$$\text{Kangrun chemiluminescent AMH} = a5+b5*\text{Beckmann AMH}$$

if the Beckman AMH value is >1 ng/mL and ≤ 4 ng/mL, then g1=1, g2=0, g3=0, and the formula is:

Kangrun chemiluminescent AMH = a5+b5*Beckman AMH+c5*(Beckman AMH-1)

if the Beckman AMH value is >4 ng/mL and ≤13 ng/mL, then g1=1, g2=1, g3=0, and the formula is:

Kangrun chemiluminescent AMH = a5+b5*Beckman AMH+c5*(Beckman AMH-1)+d5*(Beckman AMH-4)

If the Beckman AMH value is >13 ng/mL, then g1=1, g2=1, g3=1, and the formula is:

Kangrun chemiluminescent AMH = a5+b5*Beckman AMH+c5*(Beckman AMH-1)+d5*(Beckman AMH-4)+e5* (Beckman AMH-13),

wherein, a5 is any value selected from -0.24101 ~ 0.26258; b5 is any value selected from a range of 0.72643 to 1.42037; c5 is any value selected from a range of -0.78107 to 0.04326; d5 is any value selected from a range of 0.29488 to 0.57880; e5 is any value selected from a range of -0.71455 to -0.47130;
preferably, the formula for converting Kangrun chemiluminescent AMH (X) to Beckman AMH (Y) is:

Beckman AMH = a6 + b6*Kangrun chemiluminescent AMH + c6*g1 (Kangrun chemiluminescent AMH-0.5) + d6*g2 (Kangrun chemiluminescent AMH-13);

in the formula, if the Kangrun chemiluminescent AMH value is $\leq$ 0.5 ng/mL, then g1=0, g2=0, and the formula is:

$$\text{Beckman AMH} = a6 + b6*\text{Kangrun chemiluminescent AMH}$$

If the Kangrun chemiluminescent AMH value is >0.5 and $\leq$13 ng/mL, then g1=1, g2=0, and the formula is:

Beckman AMH = a6 + b6*Kangrun chemiluminescent AMH + c6*(Kangrun chemiluminescent AMH-0.5)

if the Kangrun chemiluminescent AMH value is >13 ng/mL, then g1=1, g2=1, and the formula is:

Beckman AMH = a6 + b6*Kangrun chemiluminescent AMH + c6*(Kangrun chemiluminescent AMH-0.5) + d6* (Kangrun chemiluminescent AMH-13);

Among them, a6 is any value selected from a range of -0.45082 to 0.54656; b6 is any value selected from a range of -0.35329 to 1.82169; c6 is any value selected from a range of -0.63742 to 1.56010; d6 is any value selected from a range of -0.52283 to -0.22757.

10. The method according to item 6, wherein the sex hormone is anti-Miillerian hormone (AMH), wherein the first platform is Kangrun chemiluminescent AMH detection platform, and the second platform is Kangrun enzyme immunoassay (Anshlab) AMH detection platform, and spline regression is used to convert the detection results of Kangrun chemiluminescent AMH detection platform and Kangrun enzyme immunoassay (Anshlab) AMH detection platform;

preferably, the formula for converting Kangrun chemiluminescent AMH (X) to Kangrun chemiluminescent AMH (Y) is:

Kangrun enzyme immunoassay (Anshlab) AMH = a7 + b7*Kangrun chemiluminescent AMH + c7*g1*(Kangrun chemiluminescent AMH-2) + d7*g2*(Kangrun chemiluminescent AMH-4) + e7*g3*(Kangrun chemiluminescent AMH-7)

wherein, a7 is any value selected from a range of -0.31000 to 0.20149, b7 is any value selected from a range of 0.73863 to 1.10064, c7 is any value selected from a range of -0.08941 to 0.51000, d7 is any value selected from a range of 0.08121 to 0.54582, and e7 is any value selected from a range of -0.70493 to -0.42738;
further,
if Kangrun chemiluminescent AMH is $\leq$ 2 ng/mL, then g1 = 0, and the formula is:

Kangrun enzyme immunoassay (Anshlab) AMH = a7 + b7*Kangrun chemiluminescent AMH;

If Kangrun chemiluminescent AMH is >2ng/mL and $\leq$4ng/mL, then g1=1, g2=0, and the formula is:

Kangrun enzyme immunoassay (Anshlab) AMH = a7 + b7*Kangrun chemiluminescent AMH + c7*g1 (Kangrun chemiluminescent AMH-2);

if Kangrun chemiluminescent AMH is >4 ng/mL and ≤7 ng/mL, then g1=1, g2=1, and the formula is:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4)

If Kangrun chemiluminescent AMH is >7 ng/mL, then g1 = 1, g2 = 1, g3 = 1, and the formula is:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4) + e7*g3 (Kangrun chemiluminescent AMH-7).

11. The method according to item 7, wherein the sex hormone is estradiol $E_2$, wherein the first platform is the Kangrun estradiol detection platform and the second platform is the Siemens estradiol detection platform, and spline regression is used to convert the detection results of Kangrun estradiol and Siemens estradiol;

Siemens $E_2$ detection value = a8+b8*Kangrun $E_2$ detection value+c8*g1 (Kangrun $E_2$ detection value-660) + d8*g2 (Kangrun $E_2$ detection value-2030)

in the formula,

if Kangrun $E_2$ detection value is ≤ 660pmol/L, then g1=0, g2=0, and the formula is:

$$\text{Siemens } E_2 \text{ detection value} = \text{a8+b8* Kangrun } E_2 \text{ detection value}$$

if the Kangrun $E_2$ detection value is >660pmol/L and ≤2030pmol/L, then g1=1, g2=0, and the formula is:

Siemens $E_2$ detection value = a8+b8*Kangrun $E_2$ detection value + c8*(Kangrun $E_2$ detection value-660)

if Kangrun $E_2$ detection value is >2030pmol/L, then g1=1, g2=1, and the formula is:

Siemens $E_2$ detection value = a8+b8*Kangrun $E_2$ detection value+c8*(Kangrun $E_2$ detection value-660)+d8* (Kangrun $E_2$ detection value-2030)

wherein, a7 is any value selected from a range of -93.069 to -6.91207; b7 is any value selected from a range of 1.310892 to 1.569808; c7 is any value selected from a range of -0.8957 to -0.52322; d7 is any value selected from a range of -0.08505 to 0.085318.

12. A system for converting hormone data detected by different detection methods, comprising:

a submodule for acquiring a first detection value for the hormone from a first platform;
a submodule for acquiring a second detection value for the hormone from a second platform;
a submodule for fitting the first and the second detection values using Passing-Bablok regression;
a submodule for determining whether there is a systematic difference between the first and the second detection values based on the Passing-Bablok regression fitting result;
In the above submodule for determining whether there is a systematic difference between the first and the second detection values based on the Passing-Bablok regression fitting result, the following steps are performed:

if there is no systematic difference between the first and the second detection values, the first detection value or the second detection value is converted based on the fitting formula of Passing-Bablok regression;
if there is a systematic difference between the first and the second detection values, the first and the second detection values are fitted based on spline regression, and the first detection value or the second detection value is converted based on the fitting formula of spline regression.

13. The system according to item 12, wherein the determination of whether there is a systematic difference between the first and the second detection values based on the Passing-Bablok regression fitting results is made by calculating the 95% confidence interval of the intercept of the linear regression to assess the presence of a systematic difference between the first and the second detection values.

14. The system according to item 13, wherein,

the 95% confidence interval of the intercept comprises 0, indicating that there is no systematic difference between the first and the second detection values;
the 95% confidence interval of the intercept doesn't comprise 0, indicating that there is systematic difference between the first and the second detection values.

15. The system according to any one of items 12 to 14, wherein
when fitting the first and the second detection values based on the spline regression, the nodes of the spline regression are confirmed by using the intrinsic nodes and the statistical indicator.

16. The system according to item 15, wherein the statistical indicator is an AIC (Akaike Information Criterion) or a BIC (Bayesian Information Criterion) indicator.

17. The system according to any one of items 12 to 16, wherein the hormone is a sex hormone, and the sex hormone is selected from hypothalamic-pituitary-gonadal axis hormones, including hypothalamic hormones, pituitary prolactin (PRL), follicle-stimulating hormone (FSH), luteinizing hormone (LH), anti-Müllerian hormone (AMH), inhibin B, estradiol ($E_2$), testosterone (T), dehydroepiandrosterone sulfate (DHEA-S), estriol ($E_3$), progesterone (P), preferably anti-Miillerian hormone (AMH) or estradiol ($E_2$).

18. The system according to item 17, wherein the sex hormone is anti-Müllerian hormone (AMH), and when the first platform is the Roche AMH detection platform and the second platform is the Kangrun chemiluminescent AMH detection platform, Passing-Bablok regression is used to convert the detection results of the Roche AMH detection platform and the Kangrun chemiluminescent AMH detection platform;

preferably, the formula for converting Kangrun chemiluminescent AMH (X) to Roche AMH (Y) is:

$$Roche\ AMH = a1 + b1 * Kangrun\ chemiluminescent\ AMH;$$

wherein a1 is any value selected from a range of -0.0035 to 0.0303; b1 is any value selected from a range of 0.85 to 0.8844;
preferably, the formula for converting Roche AMH (X) to Kangrun chemiluminescent AMH (Y) is:

$$Kangrun\ chemiluminescent\ AMH = a2 + b2 * Roche\ AMH;$$

wherein a2 is any value selected from a range of -0.0356 to 0.00393; b2 is any value selected from a range of 1.1307 to 1.1765.

19. The system according to item 17, wherein the sex hormone is anti-Miillerian hormone (AMH), and when the first platform is the Beckman AMH detection platform and the second platform is the Roche AMH detection platform, spline regression is used to convert the detection results of the Beckman AMH detection platform and the Roche AMH detection platform;

preferably, the formula for converting Roche AMH (X) to Beckman AMH (Y) is:

Beckman AMH = a3 + b3*Roche AMH + c3*g1 (Roche AMH-1) + d3*g2 (Roche AMH-9),

in the formula, if Roche AMH is $\leq$ 1 ng/mL, then g1 = 0, g2 = 0, and the formula is:

$$Beckman\ AMH = a3 + b3 * Roche\ AMH$$

if Roche AMH is >1 ng/mL and $\leq$9 ng/mL, then g1=1, g2=0, and the formula is:

$$Beckman\ AMH = a3 + b3 * Roche\ AMH + c3 * (Roche\ AMH-1)$$

if Roche AMH is >9 ng/mL, then g1=1, g2=1, and the formula is:

Beckman AMH = a3 + b3*Roche AMH + c3*(Roche AMH-1) + d3*(Roche AMH-9)

wherein a3 is any value selected from a range of -0.28809 to 0.30461; b3 is any value selected from a range of 0.665236 to 1.430824; c3 is any value selected from a range of 0.073606 to 0.900514; d3 is any value selected from a range of -0.70344 to -0.42116;
preferably, the formula for converting Beckman AMH (X) to Roche AMH (Y) is:

Roche AMH = a4 + b4*Beckmann AMH + c4*g1 (Beckmann AMH-0.5) + d4*g2 (Beckmann AMH-1)

In the formula, if the Beckman AMH value is $\leq$ 0.5 ng/mL, then g1=0, g2=0, and the formula is:

$$\text{Roche AMH} = a4 + b4*\text{Beckmann AMH}$$

if the Beckman AMH value is >0.5 ng/mL and $\leq$1 ng/mL, then g1=1, g2=0, and the formula is:

$$\text{Roche AMH} = a4 + b4*\text{Beckmann AMH} + c4*(\text{Beckmann AMH} - 0.5)$$

if the Beckman AMH value is > 1 ng/mL, then g1 = 1, g2 = 1, and the formula is:

Roche AMH = a4 + b4*Beckmann AMH + c4*(Beckmann AMH-0.5) + d4*(Beckmann AMH-1);

a4 is any value selected from a range of -0.42466 to 0.41822; b4 is any value selected from a range of -0.11202 to 2.17363; c4 is any value selected from a range of -1.96122 to 1.21734; d4 is any value selected from a range of -0.49419 to 0.66553.

20. The system according to item 17, wherein the sex hormone is anti-Müllerian hormone (AMH), wherein when the first platform is the Beckman AMH detection platform and the second platform is the Kangrun chemiluminescent AMH detection platform, spline regression is used to convert the detection results of the Beckman AMH detection platform and the Kangrun chemiluminescent AMH detection platform;

preferably, the formula for converting Beckman AMH (X) to Kangrun chemiluminescent AMH (Y) is:

Kangrun chemiluminescent AMH = a5+b5*Beckman AMH+c5*g1(Beckman AMH-1)+d5*g2(Beckman AMH-4)+e5*g3(Beckman AMH-13)

in the formula, if the Beckman AMH value is $\leq$ 1 ng/mL, then g1=0, g2=0, g3=0, and the formula is:

$$\text{Kangrun chemiluminescent AMH} = a5+b5*\text{Beckmann AMH}$$

if the Beckman AMH value is >1 ng/mL and $\leq$4 ng/mL, then g1=1, g2=0, g3=0, and the formula is:

Kangrun chemiluminescent AMH = a5+b5*Beckman AMH+c5*(Beckman AMH-1)

if the Beckman AMH value is >4 ng/mL and $\leq$13 ng/mL, then g1=1, g2=1, g3=0, and the formula is:

Kangrun chemiluminescent AMH = a5+b5*Beckman AMH+c5*(Beckman AMH-1)+d5*(Beckman AMH-4)

if the Beckman AMH value is >13 ng/mL, then g1=1, g2=1, g3=1, and the formula is:

Kangrun chemiluminescent AMH = a5+b5*Beckman AMH+c5*(Beckman AMH-1)+d5*(Beckman AMH-4)+e5* (Beckman AMH-13),

wherein, a5 is any value selected from -0.24101 ~ 0.26258; b5 is any value selected from a range of 0.72643 to 1.42037; c5 is any value selected from a range of -0.78107 to 0.04326; d5 is any value selected from a range of 0.29488 to 0.57880; e5 is any value selected from a range of -0.71455 to -0.47130;
preferably, the formula for converting Kangrun chemiluminescent AMH (X) to Beckman AMH (Y) is:

Beckman AMH = a6 + b6*Kangrun chemiluminescent AMH + c6*g1 (Kangrun chemiluminescent AMH-0.5) + d6*g2 (Kangrun chemiluminescent AMH-13);

in the formula, if the Kangrun chemiluminescent AMH value is ≤ 0.5 ng/mL, then g1=0, g2=0, and the formula is:

$$\text{Beckman AMH} = \text{a6} + \text{b6*Kangrun chemiluminescent AMH}$$

if the Kangrun chemiluminescent AMH value is >0.5 and ≤13 ng/mL, then g1=1, g2=0, and the formula is:

Beckman AMH = a6 + b6*Kangrun chemiluminescent AMH + c6*(Kangrun chemiluminescent AMH-0.5)

if the Kangrun chemiluminescent AMH value is >13 ng/mL, then g1=1, g2=1, and the formula is:

Beckman AMH = a6 + b6*Kangrun chemiluminescent AMH + c6*(Kangrun chemiluminescent AMH-0.5) + d6* (Kangrun chemiluminescent AMH-13);

wherein, a6 is any value selected from a range of -0.45082 to 0.54656; b6 is any value selected from a range of -0.35329 to 1.82169; c6 is any value selected from a range of -0.63742 to 1.56010; d6 is any value selected from a range of -0.52283 to -0.22757.

21. The system according to item 17, wherein the sex hormone is anti-Müllerian hormone (AMH), wherein when the first platform is Kangrun chemiluminescent AMH detection platform, and the second platform is Kangrun enzyme immunoassay (Anshlab) AMH detection platform, and spline regression is used to convert the detection results of Kangrun chemiluminescent AMH detection platform and Kangrun enzyme immunoassay (Anshlab) AMH detection platform;

preferably, the formula for converting Kangrun chemiluminescent AMH (X) to Kangrun chemiluminescent AMH (Y) is:

Kangrun enzyme immunoassay (Anshlab) AMH = a7 + b7*Kangrun chemiluminescent AMH + c7*g1*(Kangrun chemiluminescent AMH-2) + d7*g2*(Kangrun chemiluminescent AMH-4) + e7*g3*(Kangrun chemiluminescent AMH-7)

wherein, a7 is any value selected from a range of -0.31000 to 0.20149, b7 is any value selected from a range of 0.73863 to 1.10064, c7 is any value selected from a range of -0.08941 to 0.51000, d7 is any value selected from a range of 0.08121 to 0.54582, and e7 is any value selected from a range of -0.70493 to -0.42738;
further,
if Kangrun chemiluminescent AMH is ≤ 2 ng/mL, then g1 = 0, and the formula is:

Kangrun enzyme immunoassay (Anshlab) AMH = a7 + b7*Kangrun chemiluminescent AMH;

if Kangrun chemiluminescent AMH is >2 ng/mL and ≤4 ng/mL, then g1=1, g2=0, and the formula is:

Kangrun enzyme immunoassay (Anshlab) AMH = a7 + b7*Kangrun chemiluminescent AMH + c7*g1 (Kangrun chemiluminescent AMH-2);

if Kangrun chemiluminescent AMH is >4 ng/mL and ≤7 ng/mL, then g1=1, g2=1, and the formula is:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4)

if Kangrun chemiluminescent AMH is >7 ng/mL, then g1 = 1, g2 = 1, g3 = 1, and the formula is:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4) + e7*g3 (Kangrun chemiluminescent AMH-7).

22. The system according to item 17, wherein the sex hormone is estradiol $E_2$, wherein when the first platform is the Kangrun estradiol detection platform and the second platform is the Siemens estradiol detection platform, spline regression is used to convert the detection results of Kangrun estradiol and Siemens estradiol;

Siemens $E_2$ detection value = a8+b8*Kangrun $E_2$ detection value+c8*g1 (Kangrun $E_2$ detection value-660) +d8*g2 (Kangrun $E_2$ detection value-2030);

in the formula,

if Kangrun $E_2$ detection value is $\leq$ 660pmol/L, then g1=0, g2=0, and the formula is:

$$\text{Siemens } E_2 \text{ detection value} = a8+b8* \text{ Kangrun } E_2 \text{ detection value}$$

if the Kangrun $E_2$ detection value is >660pmol/L and $\leq$2030pmol/L, then g1=1, g2=0, and the formula is:

Siemens $E_2$ detection value = a8+b8*Kangrun $E_2$ detection value + c8*(Kangrun $E_2$ detection value - 660)

if the Kangrun $E_2$ detection value is >2030pmol/L, then g1 = 1, g2 = 1, and the formula is:

Siemens $E_2$ detection value = a8+b8*Kangrun $E_2$ detection value+c8*(Kangrun $E_2$ detection value-660)+d8* (Kangrun $E_2$ detection value-2030)

wherein, a7 is any value selected from a range of -93.069 to -6.91207; b7 is any value selected from a range of 1.310892 to 1.569808; c7 is any value selected from a range of -0.8957 to -0.52322; d7 is any value selected from a range of -0.08505 to 0.085318.

**EFFECTS**

Through the method and system of the present application, the serum hormone detection values obtained by different hormone detection methods can be converted and calculated, this allows patients to easily standardize hormone detection data obtained from different hospitals or testing institutions, that is, results of the same hormone detection items from different testing systems in different hospitals can be mutually recognized through certain formula conversion. Through this data conversion, on one hand, it can reduce the cost of sex hormone detections for patients visiting different hospitals or testing institutions; on the other hand, it facilitates the promotion and application of artificial intelligence software based on data from a certain testing platform to different testing platforms for the same index item, contributing to the subsequent development of digital medicine. For example, our team has established various digital medical applications using the reproductive medicine big data from Peking University Third Hospital, including tools for assessing ovarian reserve function, assessing the number of oocytes, screening for PCOS, and calculating the starting and adjusting doses of exogenous FSH. These tools all require the input of AMH levels, but our tools are only based on the prediction of the Kangrun chemiluminescent AMH hormone levels of the Kangrun Anshlab platform; if they need to be applied to different AMH detection brands, it requires the application of this invention for conversion, which can reduce the cost of hormone detection for patients and ensure the accuracy of digital medical tools.

**BRIEF DESCRIPTION OF THE DRAWINGS**

Various other advantages and benefits of the present application will become apparent to those of ordinary skill in the art by reading the detailed description of the preferred specific embodiments provided below. The accompany drawings of the specification are intended solely for illustrating the preferred embodiments and are not considered limitations of the present application. Obviously, the accompany drawings described below are merely some embodiments of the present application. For ordinary technicians in the field, other accompany drawings can also be obtained based on these accompany drawings without exerting creative work. Also, throughout the accompany drawings, identical parts are denoted by the same reference numerals.

Fig. 1A, Fig. 1B, and Fig. 1C respectively show the fitting results of Roche AMH and Kangrun AMH, the consistency of the detection results of Roche AMH and Kangrun AMH, and the ridge plot of Kangrun AMH and Roche AMH.

Fig. 2 shows the fitting results using Deming regression and Passing-Bablok regression for the $E_2$ results.

Fig. 3 shows the Bland-Altman plot of the consistency between Kangrun and Siemens for the $E_2$ results.

Fig. 4 shows the ridge plot of Siemens $E_2$ detection value-Kangrun $E_2$ detection value.

Fig. 5 shows the comparison of predictions using Passing-Bablok regression and spline regression for the E2 platform conversion.

Fig. 6 shows the number of nodes in the linear spline regression of the AMH detection results between Kangrun chemiluminescent AMH test and Kangrun enzyme immunoassay (Anshlab) AMH test.

Fig. 7 shows the grouping of data from Kangrun chemiluminescent AMH test and Kangrun enzyme immunoassay (Anshlab) AMH test.

Fig. 8 shows the AMH data of Kangrun Enzyme immunoassay (Anshlab) calculated using the platform conversion formula.

Fig. 9 shows the AMH data of Kangrun Enzyme immunoassay (Anshlab) calculated using the platform conversion formula for the low value (<7ng/mL) data.

## DETAILED DESCRIPTION

Specific embodiments of the present application will be described in more details below. However, it should be understood that the present application can be implemented in various forms and should not be limited to the embodiments set forth herein. On the contrary, these embodiments are provided to enable a more thorough understanding of the present application and to fully convey the scope of the present application to those skilled in the art.

It should be noted that certain terms are used in the specification and claims to refer to specific components. Those skilled in the art should understand that they may use different terms to refer to the same component. This specification and the claims do not use differences in terminology as a way to distinguish components, but rather use differences in functionality of components as a criterion for differentiation. As mentioned in the entire specification and claims, " comprising" or "including" is an open-ended term and thus should be interpreted as "comprising, but not limited to". The subsequent description of the specification describes preferred embodiments of the present application, but the description is intended to illustrate general principles of the specification and is not intended to limit the scope of the application. The protection scope of the present application shall be defined by the appended claims.

Passing-Bablok regression is primarily used for fitting the consistency of the linear relationship between the dependent variable and the independent variable, assuming both the dependent and independent variables are random. The idea of Passing-Bablok regression is to calculate the slope of any two points and finally take the median of all slopes as the estimated slope value. Passing-Bablok regression primarily detects the difference between the intercept and 0 and the difference between the slope and 1 to determine whether the two methods are statistically consistent.

Spline regression is a kind of linear regression. Unlike linear regression, which fits a linear relationship across all data, spline regression divides the data into segments and fits a linear relationship within each segment, therefore, spline regression is a piecewise polynomial function that smoothly connects at nodes. Spline regression requires determining the number of nodes, their values, as well as the form of the fitting curve within each segment, which can be a linear term (linear), quadratic term, cubic term, etc., depending on the actual data.

The determination of nodes in the spline regression process typically prioritizes the intrinsic nodes already confirmed within the field, with statistical indicators serving as secondary considerations. When there are relatively clear nodes in the field based on the characteristics of the data itself in a professional sense (i.e., intrinsic nodes based on data characteristics), one should first determine based on these intrinsic nodes; when there is no clear intrinsic node in the field, consider making judgments based on statistical indicators. Commonly used indicators include AIC (Akaike Information Criterion) and BIC (Bayesian Information Criterion), which are standards for evaluating model performance; the lower the AIC or BIC, the better the model's fitting effect.

The present application provides a method for converting hormone data detected by different detection methods, comprising:

step 1: obtaining a first detection value for the hormone from a first platform;

step 2: obtaining a second detection value for the hormone from a second platform;

step 3: using Passing-Bablok regression to fit the first and the second detection values;

step 4: determining whether there is a systematic difference between the first and the second detection values based on the Passing-Bablok regression fitting results;

If there is no systematic difference between the first and the second detection values, the first detection value or the second detection value is converted based on the fitting formula of Passing-Bablok regression.

if there is a systematic difference between the first and the second detection values, the first and the second detection values are fitted based on the spline regression, and the first detection value or the second detection value is converted based on the fitting formula of spline regression.

Specifically, the order of step 1 and step 2 is not fixed.

Through the method of the present application, the serum hormone detection values obtained by different hormone detection methods can be converted and calculated, this allows patients to easily standardize hormone detection data obtained from different hospitals or testing institutions, that is, results of the same hormone detection items from different testing systems in different hospitals can be mutually recognized through certain formula conversion. Through this data conversion, on one hand, it can reduce the cost of sex hormone detections for patients visiting different hospitals or testing institutions; on the other hand, it facilitates the promotion and application of artificial intelligence software based on data from a certain testing platform to different testing platforms for the same index item, contributing to the subsequent development of digital medicine.

In the present application, the determination of whether there is a systematic difference between the first and the second detection values based on the Passing-Bablok regression fitting results is made by calculating the 95% confidence interval of the intercept of the linear regression result to assess the presence of a systematic difference between the first and second detection values.

A confidence interval refers to an estimated interval of a population parameter constructed from a sample statistic. In statistics, a confidence interval of a probability sample is an interval estimate for a certain population parameter of the sample. A confidence interval represents the degree to which the true value of a parameter is likely to lie around the measured results. The confidence interval provides the degree of reliability for the measured value of the measured parameter, i.e., the "probability" required earlier. This probability is referred to as the confidence level, and a confidence interval at 0.95 confidence level can also be expressed as a 95% confidence interval.

Specifically, the 95% confidence interval of the intercept includes 0, indicating no systematic difference between the first and the second detection values;

specifically, the 95% confidence interval of the intercept does not include 0, indicating the presence of systematic difference between the first and the second detection values.

In the present application, when fitting the first and the second detection values based on spline regression, intrinsic nodes and statistical indicators are utilized to confirm the nodes of the spline regression.

Specifically, the statistical indicator is an AIC (Akaike Information Criterion) or a BIC (Bayesian Information Criterion) indicator.

Akaike Information Criterion (AIC) is one of the commonly used criteria for comparing the goodness-of-fit of multiple regression models with different numbers of explanatory variables. This criterion mandates that an explanatory variable be added to the original model only if the added explanatory variable reduces the AIC value. If the added explanatory variable has no explanatory power, it does not significantly reduce the residual sum of square, yet increase the number of parameters to be estimated, which may lead to an increase in the AIC value.

The Bayesian Information Criterion, also known as the Bayesian Decision Criterion, is a basic method in statistical pattern recognition. The Bayesian decision Criterion takes into account both the probability of occurrence of various reference populations and the magnitude of losses caused by misjudgment, and has strong discriminatory ability. Bayesian methods are more suitable for the following situations:

(1) the number (capacity) of samples (subsamples) is not large enough, hence the statistical theory of large subsamples is not applicable;

(2) the experiment is hereditary, which, in statistical terms, means there must be prior information before the experiment.

In the present application, the hormone is a sex hormone, preferably the sex hormone is selected from hypothalamic-pituitary-gonadal axis hormones, including hypothalamic hormones, pituitary prolactin (PRL), follicle-stimulating hormone (FSH), luteinizing hormone (LH), anti-Miillerian hormone (AMH), inhibin B, estradiol ($E_2$), testosterone (T), dehydroepiandrosterone sulfate (DHEA-S), estriol ($E_3$), and progesterone (P).

Specifically, the hormone is anti-Miillerian hormone (AMH) or estradiol ($E_2$).

In the present application, the first platform can be Roche AMH detection platform, Kangrun chemiluminescent AMH detection platform, Beckman AMH detection platform, Kangrun enzyme immunoassay (Anshlab) AMH detection platform, Kangrun estradiol detection platform and Siemens estradiol detection platform.

In the present application, the second platform can be Roche AMH detection platform, Kangrun chemiluminescent AMH detection platform, Beckman AMH detection platform, Kangrun enzyme immunoassay (Anshlab) AMH detection platform, Kangrun estradiol detection platform and Siemens estradiol detection platform.

Specifically, when the first platform is the Roche AMH detection platform, the second platform can be the Kangrun chemiluminescent AMH detection platform, the Beckman AMH detection platform, the Kangrun enzyme immunoassay (Anshlab) AMH detection platform, the Kangrun estradiol detection platform and the Siemens estradiol detection platform.

Specifically, when the first platform is the Kangrun chemiluminescent AMH detection platform, the second platform can be the Roche AMH detection platform, the Beckman AMH detection platform, the Kangrun enzyme immunoassay (Anshlab) AMH detection platform, the Kangrun estradiol detection platform and the Siemens estradiol detection platform.

Specifically, when the first platform is the Beckman AMH detection platform, the second platform can be the Roche AMH detection platform, the Kangrun chemiluminescent AMH detection platform, the Kangrun enzyme immunoassay (Anshlab) AMH detection platform, the Kangrun estradiol detection platform and the Siemens estradiol detection platform.

Specifically, when the first platform is the Kangrun enzyme immunoassay (Anshlab) AMH detection platform, the second platform can be the Roche AMH detection platform, the Kangrun chemiluminescent AMH detection platform, the Beckman AMH detection platform, the Kangrun estradiol detection platform and the Siemens estradiol detection platform.

Specifically, when the first platform is the Kangrun estradiol detection platform, the second platform can be the Roche AMH detection platform, the Kangrun chemiluminescent AMH detection platform, the Beckman AMH detection platform, the Kangrun enzyme immunoassay (Anshlab) AMH detection platform and the Siemens estradiol detection platform.

Specifically, when the first platform is the Siemens estradiol detection platform, the second platform can be the Roche AMH detection platform, the Kangrun chemiluminescent AMH detection platform, the Beckman AMH detection platform, the Kangrun enzyme immunoassay (Anshlab) AMH detection platform, the Kangrun estradiol detection platform and the Siemens estradiol detection platform.

In the present application, the sex hormone is anti-Müllerian hormone (AMH), wherein when the first platform is the Roche AMH detection platform and the second platform is the Kangrun chemiluminescent AMH detection platform, Passing-Bablok regression is used to convert the detection results of the Roche AMH detection platform and the Kangrun chemiluminescent AMH detection platform;

Specifically, the formula for converting Kangrun chemiluminescent AMH (X) to Roche AMH (Y) is:

$$\text{Roche AMH} = a1 + b1 \ast \text{Kangrun chemiluminescent AMH};$$

wherein a1 is any value selected from a range of -0.0035 to 0.0303; b1 is any value selected from a range of 0.85 to 0.8844;

further, a1 can be -0.0035, -0.0015, 0, 0.001, 0.002, 0.003, 0.004, 0.005, 0.006, 0.007, 0.008, 0.009, 0.010, 0.012, 0.014, 0.016, 0.018, 0.020, 0.022, 0.024, 0.026, 0.028, 0.030 or 0.0303.

b1 can be 0.85, 0.86, 0.87, 0.88, 0.881, 0.882, 0.883, 0.884, 0.8841, 0.8842, 0.8843, and 0.8844.

Specifically, the formula for converting Roche AMH (X) to Kangrun chemiluminescent AMH (Y) is:

$$\text{Kangrun chemiluminescent AMH} = a2 + b2 \ast \text{Roche AMH};$$

wherein a2 is any value selected from a range of -0.0356 to 0.00393; b2 is any value selected from a range of 1.1307 to 1.1765.

Further, a2 can be -0.0356, -0.0366, -0.0376, -0.0386 or -0.0393.

b2 can be 1.1307, 1.1407, 1.1507, 1.1607, 1.1707 or 1.1765.

In the present application, the sex hormone is anti-Müllerian hormone (AMH), wherein when the first platform is the Beckman AMH detection platform and the second platform is the Roche AMH detection platform, spline regression is used to convert the detection results of the Beckman AMH detection platform and the Roche AMH detection platform;

Specifically, the formula for converting Roche AMH (X) to Beckman AMH (Y) is:

Beckman AMH = a3 + b3*Roche AMH + c3*g1 (Roche AMH-1) + d3*g2 (Roche AMH-9),

in the formula, if Roche AMH is $\leq$ 1 ng/mL, then g1 = 0, g2 = 0, and the formula is:

$$\text{Beckman AMH} = a3 + b3*\text{Roche AMH};$$

if Roche AMH is > 1 ng/mL and ≤9 ng/mL, then g1=1, g2=0, and the formula is:

$$\text{Beckman AMH} = a3 + b3*\text{Roche AMH} + c3*(\text{Roche AMH-1});$$

if Roche AMH is >9 ng/mL, then g1=1, g2=1, and the formula is:

Beckman AMH = a3 + b3*Roche AMH + c3*(Roche AMH-1) + d3*(Roche AMH-9);

wherein a3 is any value selected from a range of -0.28809 to 0.30461; b3 is any value selected from a range of 0.665236 to 1.430824; c3 is any value selected from a range of 0.073606 to 0.900514; d3 is any value selected from a range of -0.70344 to -0.42116;
further, a3 can be -0.28809, -0.18809, 0, 0.18809, 0.28809 or 0.30461.

b3 can be 0.665236, 0.765236, 0.865236, 0.965236, 1 or 1.430824.
c3 can be 0.073606, 0.083606, 0.093606, 0.103606, 0.203606, 0.303606, 0.403606, 0.503606, 0.603606, 0.703606, 0.803606 or 0.900514.
d3 can be -0.70344, -0.60344, -0.50344, or -0.42116.
Specifically, the formula for converting Beckman AMH (X) to Roche AMH (Y) is:

Roche AMH = a4 + b4*Beckmann AMH + c4*g1 (Beckmann AMH-0.5) + d4*g2 (Beckmann AMH-1);

in the formula, if the Beckman AMH value is ≤ 0.5 ng/mL, then g1=0, g2=0, and the formula is:

$$\text{Roche AMH} = a4 + b4*\text{Beckmann AMH};$$

if the Beckman AMH value is >0.5 ng/mL and ≤1 ng/mL, then g1=1, g2=0, and the formula is:

$$\text{Roche AMH} = a4 + b4*\text{Beckmann AMH} + c4*(\text{Beckmann AMH - 0.5});$$

if the Beckman AMH value is > 1 ng/mL, then g1 = 1, g2 = 1, and the formula is:

Roche AMH = a4 + b4*Beckmann AMH + c4*(Beckmann AMH-0.5) + d4*(Beckmann AMH-1);

a4 is any value selected from a range of -0.42466 to 0.41822; b4 is any value selected from a range of -0.11202 to 2.17363; c4 is any value selected from a range of -1.96122 to 1.21734; d4 is any value selected from a range of -0.49419 to 0.66553;
further, a4 can be -0.42466, -0.32466, -0.22466, -0.12466, -0.02466, 0, 0.12466, 0.22466, 0.32466 or 0.41822.

b4 can be -0.11202, -0.01202, 0, 0.11202, 0.21202, 0.31202, 0.41202, 0.51202, 0.61202, 0.71202, 0.81202, 0.91202, 1.11202, 1.21202, 1.31202, 1.41202, 1.51202, 1.61202, 1.71202, 1.81202, 1.91202, 2.01202, 2.11202 or 2.17363.
c4 can be -1.96122, -1.86122, -1.76122, -1.66122, -1.56122, -1.46122, -1.36122, -1.26122, -1.16122, -1.06122, -0.96122, -0.86122, -0.76122, -0.66122, -0.56122, -0.46122, -0.36122, -0.26122, -0.16122, -0.06122, 0, 0.16122, 0.26122, 0.36122, 0.46122, 0.56122, 0.66122, 0.76122, 0.86122, 0.96122, 1.06122, 1.16122 or 1.21734.
d4 can be -0.49419, -0.39419, -0.29419, -0.19419, -0.09419, 0, 0.09419, 0.19419, 0.29419, 0.39419, 0.49419, 0.59419, or 0.66553.
In the present application, the sex hormone is anti-Müllerian hormone (AMH), wherein the first platform is the Beckman AMH detection platform, and the second platform is the Kangrun chemiluminescent AMH detection platform, and spline regression is used to convert the detection results of the Beckman AMH detection platform and the Kangrun chemiluminescent AMH detection platform;

specifically, the formula for converting Beckman AMH (X) to Kangrun chemiluminescent AMH (Y) is:

Kangrun chemiluminescent AMH = a5+b5*Beckman AMH+c5*g1(Beckman AMH-1)+d5*g2(Beckman AMH-4) +e5*g3(Beckman AMH-13);

in the formula, if the Beckman AMH value is ≤ 1 ng/mL, then g1=0, g2=0, g3=0, and the formula is:

$$\text{Kangrun chemiluminescent AMH} = \text{a5+b5*Beckmann AMH};$$

if the Beckman AMH value is >1 ng/mL and ≤4 ng/mL, then g1=1, g2=0, g3=0, and the formula is:

Kangrun chemiluminescent AMH = a5+b5*Beckman AMH+c5*(Beckman AMH-1);

if the Beckman AMH value is >4 ng/mL and ≤13 ng/mL, then g1=1, g2=1, g3=0, and the formula is:

Kangrun chemiluminescent AMH = a5+b5*Beckman AMH+c5*(Beckman AMH-1)+d5*(Beckman AMH-4);

if the Beckman AMH value is >13 ng/mL, then g1=1, g2=1, g3=1, and the formula is:

Kangrun chemiluminescent AMH = a5+b5*Beckman AMH+c5*(Beckman AMH-1)+d5*(Beckman AMH-4)+e5* (Beckman AMH-13),

wherein, a5 is any value selected from -0.24101 ~ 0.26258; b5 is any value selected from a range of 0.72643 to 1.42037; c5 is any value selected from a range of -0.78107 to 0.04326; d5 is any value selected from a range of 0.29488 to 0.57880; e5 is any value selected from a range of -0.71455 to -0.47130; further, a5 can be -0.24101, -0.14101, -0.04101, 0, 0.14101, 0.24101 or 0.26258.

b5 can be 0.72643, 0.82643, 0.92643, 1.12643, 1.22643, 1.32643 or 1.42037.
c5 can be -0.78107, -0.68107, -0.58107, -0.48107, -0.38107, -0.28107, -0.18107, -0.08107, 0 or 0.04326.
d5 can be 0.29488, 0.39488, 0.49488 or 0.57880.
e5 can be -0.71455, -0.61455, -0.51455 or -0.47130.
Specifically, the formula for converting Kangrun chemiluminescent AMH (X) to Beckmann AMH (Y) is:

Beckman AMH = a6 + b6*Kangrun chemiluminescent AMH + c6*g1 (Kangrun chemiluminescent AMH-0.5) + d6*g2 (Kangrun chemiluminescent AMH-13);

in the formula, if the Kangrun chemiluminescent AMH value is ≤ 0.5 ng/mL, then g1=0, g2=0, and the formula is:

$$\text{Beckman AMH} = \text{a6+b6*Kangrun chemiluminescent AMH};$$

if the Kangrun chemiluminescent AMH value is >0.5 and ≤13 ng/mL, then g1=1, g2=0, and the formula is:

Beckman AMH = a6 + b6*Kangrun chemiluminescent AMH + c6*(Kangrun chemiluminescent AMH-0.5);

if the Kangrun chemiluminescent AMH value is >13 ng/mL, then g1=1, g2=1, and the formula is:

Beckman AMH = a6 + b6*Kangrun chemiluminescent AMH + c6*(Kangrun chemiluminescent AMH-0.5) + d6*(Kangrun chemiluminescent AMH-13);

wherein, a6 is any value selected from a range of -0.45082 to 0.54656; b6 is any value selected from a range of -0.35329 to 1.82169; c6 is any value selected from a range of -0.63742 to 1.56010; d6 is any value selected from a range of -0.52283 to -0.22757.
Further, a6 can be -0.45082, -0.35082, -0.25082, -0.15082, -0.05082, 0, 0.05082, 0.15082, 0.25082, 0.35082, 0.45082 or 0.54656.
b6 can be -0.35329, -0.25329, -0.15329, -0.05329, 0, 0.05329, 0.15329, 0.25329, 0.35329, 0.45329, 0.55329, 0.65329, 0.75329, 0.85329, 0.95329, 1.05329, 1.15329, 1.25329, 1.35329, 1.45329, 1.55329, 1.16329, 1.17329 or 1.82169.

c6 can be -0.63742, -0.53742, -0.43742, -0.33742, -0.23742, -0.13742, -0.03742, 0, 0.03742, 0.13742, 0.23742, 0.33742, 0.43742, 0.53742, 0.63742, 0.73742, 0.83742, 0.93742, 1.03742, 1.13742, 1.23742, 1.33742, 1.43742, or 1.56010.

d6 can be -0.52283, -0.42283, -0.32283 or -0.22757.

In the present application, the sex hormone is anti-Müllerian hormone (AMH), wherein the sex hormone is anti-Müllerian hormone (AMH), wherein the first platform is Kangrun chemiluminescent AMH detection platform, and the second platform is Kangrun enzyme immunoassay (Anshlab) AMH detection platform, spline regression is used to convert the detection results of Kangrun chemiluminescent AMH detection platform and Kangrun enzyme immunoassay (Anshlab) AMH detection platform;

specifically, the formula for converting Kangrun chemiluminescent AMH (X) to Kangrun chemiluminescent AMH (Y) is:

Kangrun enzyme immunoassay (Anshlab) AMH = a7+b7*Kangrun chemiluminescent AMH + c7*g1* (Kangrun chemiluminescent AMH-2) + d7*g2* (Kangrun chemiluminescent AMH-4) + e7*g3*(Kangrun chemiluminescent AMH-7);

wherein, a7 is any value selected from a range of -0.31000 to 0.20149, b7 is any value selected from a range of 0.73863 to 1.10064, c7 is any value selected from a range of -0.08941 to 0.51000, d7 is any value selected from a range of 0.08121 to 0.54582, and e7 is any value selected from a range of -0.70493 to -0.42738;

further, a7 can be -0.31000, -0.21000, -0.11000, -0.01000, 0, 0.01000, 0.11000 or 0.20149.

b7 can be 0.73863, 0.83863, 0.93863, 1.03863 or 1.10064.

c7 can be -0.08941, 0, 0.08941, 0.18941, 0.28941, 0.38941, 0.48941 or 0.51000.

d7 can be 0.08121, 0.18121, 0.28121, 0.38121, 0.48121 or 0.54582.

e7 can be -0.70493, -0.60493, -0.50493, or -0.42738.

Further, if Kangrun chemiluminescent AMH is $\leq$ 2 ng/mL, then g1 = 0, and the formula is:

Kangrun enzyme immunoassay (Anshlab) AMH = a7 + b7*Kangrun chemiluminescent AMH;

if Kangrun chemiluminescent AMH is >2ng/mL and $\leq$4ng/mL, then g1=1, g2=0, and the formula is:

Kangrun enzyme immunoassay (Anshlab) AMH = a7 + b7*Kangrun chemiluminescent AMH + c7*g1 (Kangrun chemiluminescent AMH-2);

if Kangrun chemiluminescent AMH is >4 ng/mL and $\leq$7 ng/mL, then g1=1, g2=1, and the formula is:

Kangrun enzyme immunoassay (Anshlab) AMH=a7+b7*Kangrun chemiluminescent AMH + c7*g1 (Kangrun chemiluminescent AMH-2) + d7*g2 (Kangrun chemiluminescent AMH-4);

if Kangrun chemiluminescent AMH is >7 ng/mL, then g1 = 1, g2 = 1, g3 = 1, and the formula is:

Kangrun enzyme immunoassay (Anshlab) AMH = a7+b7*Kangrun chemiluminescent AMH + c7*g1 (Kangrun chemiluminescent AMH-2) + d7*g2 (Kangrun chemiluminescent AMH-4) + e7*g3 (Kangrun chemiluminescent AMH-7).

In the present application, the sex hormone is estradiol $E_2$, wherein the first platform is the Kangrun estradiol detection platform and the second platform is the Siemens estradiol detection platform, and spline regression is used to convert the detection results of Kangrun estradiol and Siemens estradiol;

Siemens $E_2$ detection value = a8+b8*Kangrun $E_2$ detection value+c8*g1 (Kangrun $E_2$ detection value-660)+d8*g2 (Kangrun $E_2$ detection value-2030);

in the formula,

if the Kangrun $E_2$ detection value is $\leq$ 660pmol/L, then g1=0, g2=0, and the formula is:

$$\text{Siemens } E_2 \text{ detection value} = a8+b8* \text{ Kangrun } E_2 \text{ detection value;}$$

if the Kangrun $E_2$ detection value is >660pmol/L and ≤2030pmol/L, then g1=1, g2=0, and the formula is:

Siemens $E_2$ detection value = a8+b8*Kangrun $E_2$ detection value + c8*(Kangrun $E_2$ detection value - 660);

if the Kangrun $E_2$ detection value is >2030pmol/L, then g1 = 1, g2 = 1, and the formula is:

Siemens $E_2$ detection value = a8+b8*Kangrun $E_2$ detection value+c8*(Kangrun $E_2$ detection value-660)+d8*(Kangrun $E_2$ detection value-2030);

wherein, a7 is any value selected from a range of -93.069 to -6.91207; b7 is any value selected from a range of 1.310892 to 1.569808; c7 is any value selected from a range of -0.8957 to -0.52322; d7 is any value selected from a range of -0.08505 to 0.085318.

Further, a7 can be -93.069, -83.069, -73.069, -63.069, -53.069, -43.069, -33.069, -23.069, -13.069 or -6.91207.

b7 can be 1.310892, 1.410892, 1.510892 or 1.569808.

c7 can be -0.8957, -0.7957, -0.6957, -0.5957 or -0.52322.

d7 can be -0.08505, -0.07505, -0.06505, -0.05505, -0.04505, -0.03505, -0.02505, -0.01505, 0, 0.01505, 0.02505, 0.03505, 0.04505, 0.05505, 0.06505, 0.07505 or 0.085318.

The present application provides a system for converting hormone data detected by different detection methods, comprising:

a submodule for acquiring a first detection value for the hormone from a first platform;
a submodule for acquiring a second detection value for the hormone from a second platform;
a submodule for fitting the first and the second detection values using Passing-Bablok regression;
a submodule for determining whether there is a systematic difference between the first and the second detection values based on the Passing-Bablok regression fitting result;

In the above submodule for determining whether there is a systematic difference between the first and the second detection values based on the Passing-Bablok regression fitting result, the following steps are performed:

if there is no systematic difference between the first and the second detection values, the first detection value or the second detection value is converted based on the fitting formula of Passing-Bablok regression,
if there is a systematic difference between the first and the second detection values, the first and the second detection values are fitted based on spline regression, and the first detection value or the second detection value is converted based on the fitting formula of spline regression.

In the present application, the determination of whether there is a systematic difference between the first and the second detection values based on the Passing-Bablok regression fitting results is made by calculating the 95% confidence interval of the intercept of the linear regression to assess the presence of a systematic difference between the first and the second detection values.

In the present application, the 95% confidence interval of the intercept comprises 0, indicating that there is no systematic difference between the first and the second detection values; the 95% confidence interval of the intercept doesn't comprise 0, indicating that there is systematic difference between the first and the second detection values.

In the present application, when fitting the first and the second detection values based on the spline regression, the nodes of the spline regression are confirmed by using the intrinsic nodes and the statistical indicator.

In the present application, the statistical indicator is the AIC (Akaike Information Criterion) or BIC (Bayesian Information Criterion) indicator.

In the present application, the hormone is a sex hormone, and the sex hormone is selected from hypothalamic-pituitary-gonadal axis hormones, including hypothalamic hormones, pituitary prolactin (PRL), follicle-stimulating hormone (FSH), luteinizing hormone (LH), anti-Miillerian hormone (AMH), inhibin B, estradiol ($E_2$), testosterone (T), dehydroepiandrosterone sulfate (DHEA-S), estriol ($E_3$), progesterone (P), preferably anti-Miillerian hormone (AMH) or estradiol ($E_2$).

In the present application, the sex hormone is anti-Müllerian hormone (AMH), and when the first platform is the Roche AMH detection platform and the second platform is the Kangrun chemiluminescent AMH detection platform, Passing-Bablok regression is used to convert the detection results of the Roche AMH detection platform and the Kangrun chemiluminescent AMH detection platform;

specifically, the formula for converting Kangrun chemiluminescent AMH (X) to Roche AMH (Y) is:

$$\text{Roche AMH} = a1 + b1*\text{Kangrun chemiluminescent AMH};$$

wherein a1 is any value selected from a range of -0.0035 to 0.0303; b1 is any value selected from a range of 0.85 to 0.8844;
further, a1 can be -0.0035, -0.0015, 0, 0.001, 0.002, 0.003, 0.004, 0.005, 0.006, 0.007, 0.008, 0.009, 0.010, 0.012, 0.014, 0.016, 0.018, 0.020, 0.022, 0.024, 0.026, 0.028, 0.030 or 0.0303.

b1 can be 0.85, 0.86, 0.87, 0.88, 0.881, 0.882, 0.883, 0.884, 0.8841, 0.8842, 0.8843, or 0.8844.

Specifically, the formula for converting Roche AMH (X) to Kangrun chemiluminescent AMH (Y) is:

$$\text{Kangrun chemiluminescent AMH} = a2 + b2*\text{Roche AMH};$$

wherein a2 is any value selected from a range of -0.0356 to 0.00393; b2 is any value selected from a range of 1.1307 to 1.1765.

Further, a2 can be -0.0356, -0.0366, -0.0376, -0.0386 or -0.0393.

b2 can be 1.1307, 1.1407, 1.1507, 1.1607, 1.1707 or 1.1765.

In the present application, the sex hormone is anti-Müllerian hormone (AMH), wherein when the first platform is the Beckman AMH detection platform and the second platform is the Roche AMH detection platform, spline regression is used to convert the detection results of the Beckman AMH detection platform and the Roche AMH detection platform;

specifically, the formula for converting Roche AMH (X) to Beckman AMH (Y) is:

Beckman AMH = a3 + b3*Roche AMH + c3*g1 (Roche AMH-1) + d3*g2 (Roche AMH-9),

in the formula, if Roche AMH is ≤ 1 ng/mL, then g1 = 0, g2 = 0, and the formula is:

$$\text{Beckman AMH} = a3 + b3*\text{Roche AMH}$$

if Roche AMH is >1 ng/mL and ≤9 ng/mL, then g1=1, g2=0, and the formula is:

$$\text{Beckman AMH} = a3 + b3*\text{Roche AMH} + c3*(\text{Roche AMH-1})$$

if Roche AMH is >9 ng/mL, then g1=1, g2=1, and the formula is:

Beckman AMH = a3 + b3*Roche AMH + c3*(Roche AMH-1) + d3*(Roche AMH-9)

wherein a3 is any value selected from a range of -0.28809 to 0.30461; b3 is any value selected from a range of 0.665236 to 1.430824; c3 is any value selected from a range of 0.073606 to 0.900514; d3 is any value selected from a range of -0.70344 to -0.42116;
further, a3 can be -0.28809, -0.18809, 0, 0.18809, 0.28809 or 0.30461.

b3 can be 0.665236, 0.765236, 0.865236, 0.965236, 1 or 1.430824.

c3 can be 0.073606, 0.083606, 0.093606, 0.103606, 0.203606, 0.303606, 0.403606, 0.503606, 0.603606, 0.703606, 0.803606 or 0.900514.

d3 can be -0.70344, -0.60344, -0.50344, or -0.42116.

Specifically, the formula for converting Beckman AMH (X) to Roche AMH (Y) is:

Roche AMH = a4 + b4*Beckmann AMH + c4*g1 (Beckmann AMH-0.5) + d4*g2 (Beckmann AMH-1);

in the formula, if the Beckman AMH value is ≤ 0.5 ng/mL, then g1=0, g2=0, and the formula is:

$$\text{Roche AMH} = a4 + b4*\text{Beckmann AMH};$$

if the Beckman AMH value is >0.5 ng/mL and ≤1 ng/mL, then g1=1, g2=0, and the formula is:

$$\text{Roche AMH} = a4 + b4*\text{Beckmann AMH} + c4*(\text{Beckmann AMH} - 0.5);$$

if the Beckman AMH value is > 1 ng/mL, then g1 = 1, g2 = 1, and the formula is:

Roche AMH = a4 + b4*Beckmann AMH + c4*(Beckmann AMH-0.5) + d4*(Beckmann AMH-1);

a4 is any value selected from a range of -0.42466 to 0.41822; b4 is any value selected from a range of -0.11202 to 2.17363; c4 is any value selected from a range of -1.96122 to 1.21734; d4 is any value selected from a range of -0.49419 to 0.66553.

further, a4 can be -0.42466, -0.32466, -0.22466, -0.12466, -0.02466, 0, 0.12466, 0.22466, 0.32466 or 0.41822.

b4 can be -0.11202, -0.01202, 0, 0.11202, 0.21202, 0.31202, 0.41202, 0.51202, 0.61202, 0.71202, 0.81202, 0.91202, 1.11202, 1.21202, 1.31202, 1.41202, 1.51202, 1.61202, 1.71202, 1.81202, 1.91202, 2.01202, 2.11202 or 2.17363.

c4 can be -1.96122, -1.86122, -1.76122, -1.66122, -1.56122, -1.46122, -1.36122, -1.26122, -1.16122, -1.06122, -0.96122, -0.86122, -0.76122, -0.66122, -0.56122, -0.46122, -0.36122, -0.26122, -0.16122, -0.06122, 0, 0.16122, 0.26122, 0.36122, 0.46122, 0.56122, 0.66122, 0.76122, 0.86122, 0.96122, 1.06122, 1.16122 or 1.21734.

d4 can be -0.49419, -0.39419, -0.29419, -0.19419, -0.09419, 0, 0.09419, 0.19419, 0.29419, 0.39419, 0.49419, 0.59419 or 0.66553.

In the present application, the sex hormone is anti-Müllerian hormone (AMH), and when the first platform is the Beckman AMH detection platform and the second platform is the Kangrun chemiluminescent AMH detection platform, spline regression is used to convert the detection results of the Beckman AMH detection platform and the Kangrun chemiluminescent AMH detection platform;

specifically, the formula for converting Beckman AMH (X) to Kangrun chemiluminescent AMH (Y) is:

Kangrun chemiluminescent AMH = a5+b5*Beckman AMH+c5*g1(Beckman AMH-1)+d5*g2(Beckman AMH-4) +e5*g3(Beckman AMH-13);

in the formula, if the Beckman AMH value is ≤ 1 ng/mL, then g1=0, g2=0, g3=0, and the formula is:

$$\text{Kangrun chemiluminescent AMH} = a5+b5*\text{Beckmann AMH};$$

if the Beckman AMH value is >1 ng/mL and ≤4 ng/mL, then g1=1, g2=0, g3=0, and the formula is:

Kangrun chemiluminescent AMH = a5+b5*Beckman AMH+c5*(Beckman AMH-1);

if the Beckman AMH value is >4 ng/mL and ≤13 ng/mL, then g1=1, g2=1, g3=0, and the formula is:

Kangrun chemiluminescent AMH = a5+b5*Beckman AMH+c5*(Beckman AMH-1)+d5*(Beckman AMH-4);

if the Beckman AMH value is >13 ng/mL, then g1=1, g2=1, g3=1, and the formula is:

Kangrun chemiluminescent AMH = a5+b5*Beckman AMH+c5*(Beckman AMH-1)+d5*(Beckman AMH-4)+e5* (Beckman AMH-13),

wherein, a5 is any value selected from -0.24101 ~ 0.26258; b5 is any value selected from a range of 0.72643 to 1.42037; c5 is any value selected from a range of -0.78107 to 0.04326; d5 is any value selected from a range of 0.29488 to 0.57880; e5 is any value selected from a range of -0.71455 to -0.47130;

further, a5 can be -0.24101, -0.14101, -0.04101, 0, 0.14101, 0.24101 or 0.26258.

b5 can be 0.72643, 0.82643, 0.92643, 1.12643, 1.22643, 1.32643 or 1.42037.

c5 can be -0.78107, -0.68107, -0.58107, -0.48107, -0.38107, -0.28107, -0.18107, -0.08107, 0 or 0.04326.

d5 can be 0.29488, 0.39488, 0.49488 or 0.57880.

e5 can be -0.71455, -0.61455, -0.51455 or -0.47130.

Specifically, the formula for converting Kangrun chemiluminescent AMH (X) to Beckmann AMH (Y) is:

Beckman AMH = a6 + b6*Kangrun chemiluminescent AMH + c6*g1 (Kangrun chemiluminescent AMH-0.5) + d6*g2 (Kangrun chemiluminescent AMH-13);

in the formula, if the Kangrun chemiluminescent AMH value is ≤ 0.5 ng/mL, then g1=0, g2=0, and the formula is:

$$\text{Beckman AMH} = \text{a6} + \text{b6*Kangrun chemiluminescent AMH};$$

if the Kangrun chemiluminescent AMH value is >0.5 and ≤13 ng/mL, then g1=1, g2=0, and the formula is:

Beckman AMH = a6 + b6*Kangrun chemiluminescent AMH + c6*(Kangrun chemiluminescent AMH-0.5);

if the Kangrun chemiluminescent AMH value is >13 ng/mL, then g1=1, g2=1, and the formula is:

Beckman AMH = a6 + b6*Kangrun chemiluminescent AMH + c6*(Kangrun chemiluminescent AMH-0.5) + d6*(Kangrun chemiluminescent AMH-13);

wherein, a6 is any value selected from a range of -0.45082 to 0.54656; b6 is any value selected from a range of -0.35329 to 1.82169; c6 is any value selected from a range of -0.63742 to 1.56010; d6 is any value selected from a range of -0.52283 to -0.22757.

further, a6 can be -0.45082, -0.35082, -0.25082, -0.15082, -0.05082, 0, 0.05082, 0.15082, 0.25082, 0.35082, 0.45082 or 0.54656.

b6 can be -0.35329, -0.25329, -0.15329, -0.05329, 0, 0.05329, 0.15329, 0.25329, 0.35329, 0.45329, 0.55329, 0.65329, 0.75329, 0.85329, 0.95329, 1.05329, 1.15329, 1.25329, 1.35329, 1.45329, 1.55329, 1.16329, 1.17329 or 1.82169.

c6 can be -0.63742, -0.53742, -0.43742, -0.33742, -0.23742, -0.13742, -0.03742, 0, 0.03742, 0.13742, 0.23742, 0.33742, 0.43742, 0.53742, 0.63742, 0.73742, 0.83742, 0.93742, 1.03742, 1.13742, 1.23742, 1.33742, 1.43742, or 1.56010.

d6 can be -0.52283, -0.42283, -0.32283 or -0.22757.

In the present application, the sex hormone is anti-Müllerian hormone (AMH), wherein when the first platform is Kangrun chemiluminescent AMH detection platform, and the second platform is Kangrun enzyme immunoassay (Anshlab) AMH detection platform, and spline regression is used to convert the detection results of Kangrun chemiluminescent AMH detection platform and Kangrun enzyme immunoassay (Anshlab) AMH detection platform;

specifically, the formula for converting Kangrun chemiluminescent AMH (X) to Kangrun chemiluminescent AMH (Y) is:

Kangrun enzyme immunoassay (Anshlab) AMH = a7 + b7* Kangrun chemiluminescent AMH + c7*g1* (Kangrun chemiluminescent AMH-2) + d7*g2* (Kangrun chemiluminescent AMH-4) + e7*g3*(Kangrun chemiluminescent AMH-7);

wherein, a7 is any value selected from a range of -0.31000 to 0.20149, b7 is any value selected from a range of 0.73863 to 1.10064, c7 is any value selected from a range of -0.08941 to 0.51000, d7 is any value selected from a range of 0.08121 to 0.54582, and e7 is any value selected from a range of -0.70493 to -0.42738;

further, a7 can be -0.31000, -0.21000, -0.11000, -0.01000, 0, 0.01000, 0.11000 or 0.20149.

b7 can be 0.73863, 0.83863, 0.93863, 1.03863 or 1.10064.

c7 can be -0.08941, 0, 0.08941, 0.18941, 0.28941, 0.38941, 0.48941 or 0.51000.

d7 can be 0.08121, 0.18121, 0.28121, 0.38121, 0.48121 or 0.54582.

e7 can be -0.70493, -0.60493, -0.50493, or -0.42738.

Further, if Kangrun chemiluminescent AMH is ≤ 2 ng/mL, then g1 = 0, and the formula is:

Kangrun enzyme immunoassay (Anshlab) AMH = a7 + b7*Kangrun chemiluminescent AMH;

if Kangrun chemiluminescent AMH is >2ng/mL and ≤4ng/mL, then g1=1, g2=0, and the formula is:

Kangrun enzyme immunoassay (Anshlab) AMH = a7 + b7*Kangrun chemiluminescent AMH + c7*g1 (Kangrun chemiluminescent AMH-2);

if Kangrun chemiluminescent AMH is >4 ng/mL and ≤7 ng/mL, then g1=1, g2=1, and the formula is:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4);

if Kangrun chemiluminescent AMH is >7 ng/mL, then g1 = 1, g2 = 1, g3 = 1, and the formula is:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4) + e7*g3 (Kangrun chemiluminescent AMH-7).

In the present application, the sex hormone is estradiol $E_2$, wherein when the first platform is the Kangrun estradiol detection platform and the second platform is the Siemens estradiol detection platform, spline regression is used to convert the detection results of Kangrun estradiol and Siemens estradiol;

Siemens $E_2$ detection value = a8+b8*Kangrun $E_2$ detection value+c8*g1 (Kangrun $E_2$ detection value-660)+d8*g2 (Kangrun $E_2$ detection value-2030);

in the formula,

if Kangrun $E_2$ detection value is ≤ 660pmol/L, then g1=0, g2=0, and the formula is:

$$\text{Siemens } E_2 \text{ detection value} = a8+b8* \text{ Kangrun } E_2 \text{ detection value;}$$

if the Kangrun $E_2$ detection value is >660pmol/L and ≤2030pmol/L, then g1=1, g2=0, and the formula is:

Siemens $E_2$ detection value = a8+b8*Kangrun $E_2$ detection value + c8*(Kangrun $E_2$ detection value - 660);

if the Kangrun $E_2$ detection value is >2030pmol/L, then g1 = 1, g2 = 1, and the formula is:

Siemens $E_2$ detection value = a8+b8*Kangrun $E_2$ detection value+c8*(Kangrun $E_2$ detection value-660)+d8*(Kangrun $E_2$ detection value-2030);

wherein, a7 is any value selected from a range of -93.069 to -6.91207; b7 is any value selected from a range of 1.310892 to 1.569808; c7 is any value selected from a range of -0.8957 to -0.52322; d7 is any value selected from a range of -0.08505 to 0.085318.

Further, a7 can be -93.069, -83.069, -73.069, -63.069, -53.069, -43.069, -33.069, -23.069, -13.069 or -6.91207.
b7 can be 1.310892, 1.410892, 1.510892 or 1.569808.
c7 can be -0.8957, -0.7957, -0.6957, -0.5957 or -0.52322.
d7 can be -0.08505, -0.07505, -0.06505, -0.05505, -0.04505, -0.03505, -0.02505, -0.01505, 0, 0.01505, 0.02505, 0.03505, 0.04505, 0.05505, 0.06505, 0.07505 or 0.085318.

**Example 1**

For the selection of samples, the distribution of AMH data from previous cases at the Reproductive Center of Peking University Third Hospital was referenced, as shown in Table 1. The AMH concentration of the collected samples was routinely tested using the Kangrun detection method. During the period from November 2021 to February 2022, the inventors of this application randomly selected 300 serum AMH samples from the Reproductive Center of Peking University Third Hospital. The samples were obtained from female patients undergoing standard in vitro fertilization (IVF). The samples were collected from the hospital's IVF center and frozen and stored at -80°C. These patients have all signed written informed consent forms, agreeing to donate their remaining serum for research use after their original blood tests. Table 1 summarizes all AMH test results detected using the Kangrun method, with units expressed in ng/mL.

Table 1. AMH distribution of samples collected in Example 1 based on Kangrun detection

| Scope | AMH (ng/mL) | Number of samples |
|---|---|---|
| Low | <0.2 | 30 |
| | 0.2-<0.4 | 30 |
| | 0.4-<0.6 | 30 |
| | 0.6-<0.8 | 30 |
| | 0.8-<1.0 | 30 |
| Medium | 1.0-<1.5 | 30 |
| | 1.5-<2.5 | 30 |
| | 2.5-<3.5 | 30 |
| High | 3.5-<15.5 | 30 |
| | ≥15.5 | 30 |

**Example 2**

For the two tubes of frozen serum of each sample obtained in Example 1, one tube was detected for AMH results using the Roche method after thawing, while the other tube was detected for results using the Kangrun chemiluminescence method and the Beckman method on the same day after thawing, thereby obtaining the AMH results from three methods (referred to as "Roche AMH", "Beckman AMH" and "Kangrun chemiluminescent AMH", respectively).

If the detection results of the serum samples collected in Example 1 exceeded the upper limit of the measurement range of each method, the reagents provided by the manufacturer of the method were used for dilution and retesting.

Roche assays were performed on the Roche Cobas e 602 instrument using the Elecsys (Roche) AMH kit; Beckman assays were performed on the Beckman DxI 800 instrument using the Beckman Access AMH kit; and Kangrun assays were performed on the Kangrun Kaeser 6600 instrument using the Kangrun Chemiluminescent AMH Kit.

Quality control of the AMH detection results detected using the three methods was performed using the quality control products (control samples) provided with each AMH kit. The difference between the measured value of the control sample and the given target value was expressed as deviation. Deviations within ± 12% and a coefficient of variation (CV) ≤ 8% were within the controllable range, and the test results were acceptable. Table 2 below showed the precision and accuracy results for each AMH assay. For each detection method, the CV values analyzed were ≤5% and the deviation is ±7%, indicating that the three methods established in the examples of the present application were stable and the detection results met the requirements.

Table 2. Comparison of the precision and accuracy of the three AMH assays

| Detection method | Control sample | The given target value (ng/mL) | Detection scope | Average detection value (ng/mL) | SD | CV (%) | Deviati on (%) |
|---|---|---|---|---|---|---|---|
| Roche method | Control 1 | 1.22 | 1.13-1.19 | 1.17 | 0.02 | 1.49 | -4.18 |
| | Control 2 | 5.92 | 5.89-6.02 | 5.89 | 0.1 | 1.78 | -0.45 |
| Beckman method | Control 1 | 1.01 | 0.86-1.02 | 0.95 | 0.04 | 4.51 | -6.24 |
| | Control 2 | 5.13 | 4.66-5.41 | 5.02 | 0.23 | 4.51 | -2.19 |
| | Control 3 | 15.2 | 13.84-15.25 | 14.37 | 0.34 | 2.38 | -5.44 |
| Kangrun che-milumin es-cence method | Control 1 | 2.39 | 2.19-2.51 | 2.37 | 0.09 | 3.67 | -0.92 |
| | Control 2 | 5.8 | 5.45-6.06 | 5.76 | 0.21 | 3.59 | -0.69 |

**Example 3**

Given the widespread use of the aforementioned three distinct AMH detection kits, it was necessary to seek an accurate method for standardizing AMH data obtained from these three methods to achieve a unified dataset.

Generally, if the AMH detection data from the Beckman method, Kangrun chemiluminescence method, and Roche method align with an overall relationship, it might be considered to use Passing-Bablok regression for pairwise data conversion among the three. Passing-Bablok regression used a non-parametric method to fit the parameters a and b in the linear equation y=a+bx. If the 95% confidence interval (CI) for the intercept a didn't not comprise 0 or the 95% confidence interval for the slope b does not comprise 1, then this indicated the presence of systematic or proportional difference between the two AMH detection results.

If the overall relationship between the detection results of two platforms was not satisfied, spline regression could be considered to find the conversion between the detection data of the two platforms. Spline regression was a piecewise polynomial function that was smoothly connected at the nodes. It could be a linear relationship (straight lines connected at the nodes), a quadratic relationship, or a cubic relationship. In spline regression, based on the relationship between the independent variable and the dependent variable, linear spline regression could be used, attempting to construct a linear regression with smooth connections at the nodes.

In Example 3, an attempt was also made to draw a Bland-Altman plot to examine the differences in systematic deviation and variance in different numerical ranges. In this example, the fitting effect of the data was assessed by the coefficient of determination ($R^2$), the corrected $R^2$, the root mean square error (RMSE), the Akaike Information Criterion (AIC) and the corrected AIC (AICc). The higher the $R^2$ and corrected $R^2$, the better the performance of the fitted model. The lower the RMSE, AIC, and AICc, the better the fitting effect. All statistical analyses in this example were performed using JMP PRO v16.0 (Cary. NC, USA).

(1) Step 1: Passing-Bablok regression among the three AMH detection results

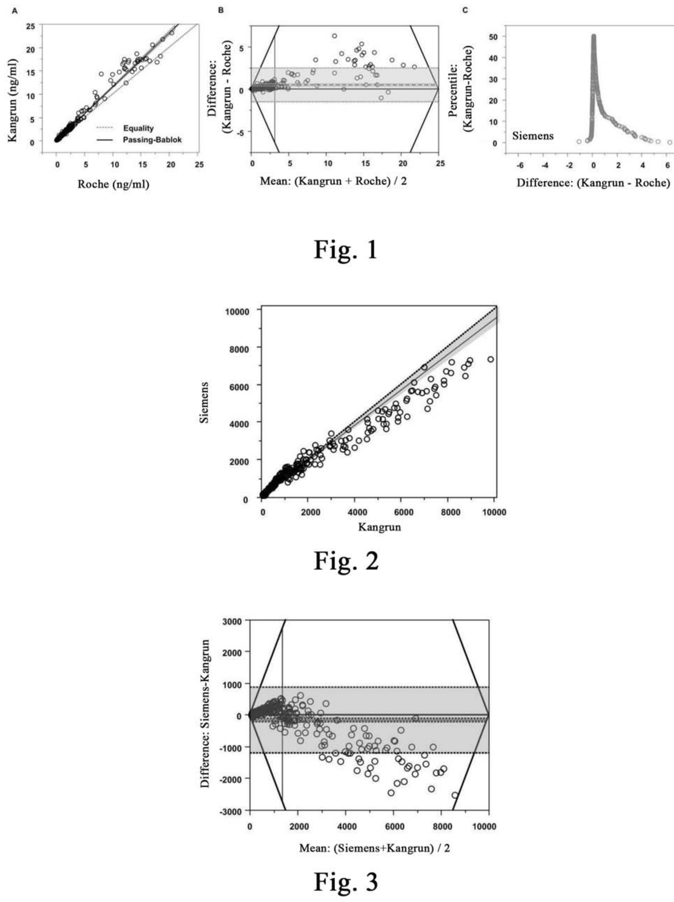

Assuming that Roche AMH and Kangrun chemiluminescent AMH conform to an overall relationship, the Passing-Bablok regression method was used to establish the linear relationship between the two detection results. Fig. 1A showed the fitting results of Roche AMH and Kangrun chemiluminescent AMH. In Fig. 1A, the dashed line reflected the theoretical fit between the two AMH assays, and the solid line represented the Passing-Bablok regression results. As can be seen in Fig. 1A, the solid line deviated from the dashed line, and this discrepancy required statistical testing.

A Bland-Altman plot was further drawn to explore the consistency of the Roche AMH and Kangrun chemiluminescent AMH detection results, as shown in Fig. 1B. The horizontal axis represented the average value of Kangrun chemiluminescent AMH and Roche AMH, and the vertical axis represented the difference between Kangrun chemiluminescent AMH and Roche AMH. The grey solid line in Fig. 1B was the reference line, i.e., the 0 value (representing the average difference of 0 between the two AMH detection results), and the central solid line and dashed line represented the mean value of AMH detected by Kangrun chemiluminescent method minus that by Roche method, along with its 95% confidence interval. The outer grey frame line (i.e., the grey area in the figure) represented the 95% distribution range of the difference (mean $\pm$ 1.96 $\times$ standard deviation), also known as the limit of agreement. Theoretically, the more consistent the two measurements were, the closer the central solid line would be to the reference line. If 95% of the points falled within the grey dashed frame and were normally distributed around the central solid line, consistency between the two AMH assays could be concluded. In this example, the number of observation points exceeding the gray line was 8.5%, and the data distribution was obviously non-normal, especially the high-value section. Therefore, based on the analysis results of the above examples, it was concluded that the two AMH measurements were inconsistent.

Further, Fig. 1C showed the ridge plot of Kangrun chemiluminescent AMH and Roche AMH. The horizontal axis of the ridge plot represented the detection value of the Kangrun method minus the detection value of the Roche method, and the vertical axis represented the percentile of the cumulative distribution. When the cumulative distribution probability was less than 50%, it was the cumulative distribution probability; when it exceeded 50%, it was 1 minus the cumulative distribution probability. If two AMH test results were consistent, they should be symmetrically distributed around 0. As can be seen from Fig. 1C, the data of "Kangrun Chemiluminescent AMH-Roche AMH" showed a right-skewed tail, indicating that most of the differences of "Kangrun Chemiluminescent AMH-Roche AMH" were greater than 0, and the long right tail suggested that some data points of "Kangrun Chemiluminescent AMH-Roche AMH" were very high. On the other hand, when the cumulative distribution was <50%, the difference between the two AMH detection methods was quite small.

The intercept for converting AMH results from the Roche method to the Kangrun chemiluminescence method was -0.0124, and its 95% confidence interval (CI) included 0, indicating no systematic difference between Kangrun AMH and Roche AMH. The slope was 1.1517 and the 95% CI did not include 1, indicating that there can be a proportional difference between the two AMH assays. The inventors of this application further employed Passing-Bablok regression to conducted pairwise conversion analysis of the other pairs among the results of three different AMH assays. The intercepts and slopes of the pairwise conversion fitting results were summarized in Table 3 below.

The inventors further standardized the pairwise data according to the fitting formula shown in Table 3. Table 4 showed the selected 30 data points, with all converted or normalized data calculated using Passing-Bablok regression (results not listed in detail).

Table 3. Conversion of AMH detection between Roche, Beckman, and Kangrun chemiluminescent methods using Passing-Bablok regression

| | Intercept (95% CI) | Slope (95% CI) |
|---|---|---|
| Conversion of Kangrun Chemiluminescent AMH to Beckman AMH | -0.1027 (-0.1264, -0.0786) | 1.1386 (1.1194, 1.1607) |
| Conversion of Kangrun chemiluminescent AMH to Roche AMH | 0.0108 (-0.0035, 0.0303) | 0.8683 (0.85, 0.8844) |
| Conversion of Beckman AMH to Roche AMH | 0.0868 (0.07, 0.1129) | 0.7632 (0.7429, 0.7789) |
| Conversion of Roche AMH to Beckman AMH | -0.1138 (-0.1515, -0.0899) | 1.3103 (1.2840, 1.3462) |
| Conversion of Roche AMH to Kangrun Chemiluminescent AMH | -0.0124 (-0.0356, 0.00393) | 1.1517 (1.1307, 1.1765) |
| Conversion of Beckman AMH to Kangrun Chemiluminescent AMH | 0.0902 (0.0702, 0.1089) | 0.8783 (0.8615, 0.8933) |

Wherein, CI stands for confidence interval.

Based on the above analysis, it can be seen that the conversion of Kangrun chemiluminescent AMH to Roche AMH, and Roche AMH to Kangrun chemiluminescent AMH, the results of Passing-Bablok regression fitting showed that the intercept included 0, so it was confirmed that the conversion formulas of Kangrun chemiluminescent AMH to Roche AMH, and Roche AMH to Kangrun chemiluminescent AMH were as follows:

the formula for converting Kangrun chemiluminescent AMH (X) to Roche AMH (Y) was:

$$\text{Roche AMH} = a1 + b1 \ast \text{Kangrun chemiluminescent AMH};$$

wherein a1 was any value selected from a range of -0.0035 to 0.0303; b1 was any value selected from a range of 0.85 to 0.8844.

The formula for converting Roche AMH (X) to Kangrun chemiluminescent AMH (Y) was:

$$\text{Kangrun chemiluminescent AMH} = a2 + b2 \ast \text{Roche AMH};$$

wherein a2 was any value selected from a range of -0.0356 to 0.00393; b2 was any value selected from a range of 1.1307 to 1.1765.

(2) Step 2: Spline regression among the three AMH detection results

The Passing-Bablok regression in (1) of Example 3 assumed that the correlation between the two AMH assays conformed to an overall relationship. However, as shown in Table 4 below and the rest data converted or normalized using Passing-Bablok regression, among the four conversions excluding the conversion of Kangrun chemiluminescent AMH to Roche AMH and Roche AMH to Kangrun chemiluminescent AMH, the intercepts of 95%CI did not include 0, and the fitting effect in the low-value section was significantly poor, indicating that they were not in an overall relationship with each other. Therefore, in Example 3(2), spline regression was further used for conversion.

Table 4. Selective display of 30 samples converted by the AMH assay using the Passing-Bablok regression method

| Sample | Original detection va lue | | | Converted or normalized values | | | | | |
|---|---|---|---|---|---|---|---|---|---|
| | Beckman AMH | Roche AMH | Kangrun che milumi nescent AMH | Conver sion of Kangr un che milumi nescent AMH to Be ckman AMH | Conver sion of Kangr un che milumi nescent AMH to Ro che AMH | Conv ersion of B eckm an A MH to Ro che A MH | Convers ion of Roche AMH t to Beck man AMH | Conversi on of R oche A MH to Kangrun chemil uminesc ent AMH | Conversion of Bec kman AMH to Kan grun che milumines cent AMH |
| 1 | 0.08 | 0.08 | 0.06 | -0.03 | 0.06 | 0.15 | 0.00 | 0.08 | 0.16 |
| 2 | 0.08 | 0.05 | 0.08 | -0.01 | 0.08 | 0.15 | -0.05 | 0.05 | 0.16 |
| 3 | 0.09 | 0.10 | 0.08 | -0.01 | 0.08 | 0.16 | 0.02 | 0.11 | 0.17 |
| 4 | 0.09 | 0.11 | 0.10 | 0.01 | 0.10 | 0.16 | 0.02 | 0.11 | 0.17 |
| 5 | 0.12 | 0.11 | 0.10 | 0.01 | 0.10 | 0.18 | 0.04 | 0.12 | 0.20 |
| 6 | 0.51 | 0.63 | 0.45 | 0.41 | 0.40 | 0.48 | 0.72 | 0.72 | 0.54 |
| 7 | 0.43 | 0.39 | 0.46 | 0.42 | 0.41 | 0.41 | 0.40 | 0.44 | 0.47 |
| 8 | 0.42 | 0.37 | 0.47 | 0.43 | 0.42 | 0.41 | 0.37 | 0.41 | 0.46 |
| 9 | 0.42 | 0.33 | 0.48 | 0.44 | 0.43 | 0.41 | 0.31 | 0.36 | 0.46 |
| 10 | 0.47 | 0.47 | 0.48 | 0.44 | 0.43 | 0.45 | 0.51 | 0.53 | 0.50 |
| 11 | 1.21 | 1.04 | 1.08 | 1.13 | 0.95 | 1.01 | 1.25 | 1.19 | 1.15 |
| 12 | 1.21 | 1.11 | 1.14 | 1.20 | 1.00 | 1.01 | 1.34 | 1.27 | 1.15 |
| 13 | 1.21 | 0.50 | 1.16 | 1.22 | 1.02 | 1.01 | 0.54 | 0.57 | 1.15 |
| 14 | 1.22 | 0.92 | 1.02 | 1.06 | 0.90 | 1.02 | 1.09 | 1.05 | 1.16 |
| 15 | 1.22 | 1.07 | 1.08 | 1.13 | 0.95 | 1.02 | 1.29 | 1.22 | 1.16 |
| 16 | 1.71 | 1.45 | 1.53 | 1.64 | 1.34 | 1.39 | 1.79 | 1.66 | 1.59 |
| 17 | 1.74 | 1.05 | 1.39 | 1.48 | 1.22 | 1.41 | 1.26 | 1.20 | 1.62 |
| 18 | 1.76 | 1.44 | 1.70 | 1.83 | 1.49 | 1.43 | 1.77 | 1.65 | 1.64 |
| 19 | 1.77 | 1.38 | 1.61 | 1.73 | 1.41 | 1.44 | 1.69 | 1.58 | 1.64 |
| 20 | 1.77 | 1.72 | 1.87 | 2.03 | 1.63 | 1.44 | 2.14 | 1.97 | 1.64 |
| 21 | 2.13 | 1.09 | 1.96 | 2.13 | 1.71 | 1.71 | 1.31 | 1.24 | 1.96 |
| 22 | 2.20 | 1.72 | 2.02 | 2.20 | 1.76 | 1.77 | 2.14 | 1.97 | 2.02 |
| 23 | 2.24 | 1.78 | 2.37 | 2.60 | 2.07 | 1.80 | 2.22 | 2.04 | 2.06 |
| 24 | 2.25 | 1.79 | 2.38 | 2.61 | 2.08 | 1.80 | 2.23 | 2.05 | 2.07 |
| 25 | 2.28 | 2.24 | 2.32 | 2.54 | 2.03 | 1.83 | 2.82 | 2.57 | 2.09 |
| 26 | 21.97 | 17.10 | 20.53 | 23.27 | 17.84 | 16.85 | 22.29 | 19.68 | 19.39 |
| 27 | 22.16 | 20.50 | 23.11 | 26.21 | 20.08 | 17.00 | 26.75 | 23.60 | 19.56 |
| 28 | 22.50 | 16.00 | 17.64 | 19.98 | 15.33 | 17.26 | 20.85 | 18.42 | 19.85 |
| 29 | 23.67 | 16.60 | 17.10 | 19.37 | 14.86 | 18.15 | 21.64 | 19.11 | 20.88 |
| 30 | 23.70 | 18.50 | 18.12 | 20.53 | 15.74 | 18.17 | 24.13 | 21.29 | 20.90 |

Taking the conversion of Roche AMH to Beckman AMH as an example, the AMH value measured by Roche was used as the independent variable x, and the AMH value measured by Beckman was used as the dependent variable y. According to the relationship between the independent variable and the dependent variable in the data of the example, a linear spline regression was further employed, that is, a linear regression with smooth connections at the nodes was constructed. In Example 3 (2), the selection of nodes was mainly based on a comprehensive consideration of the intrinsic nodes of the data in clinical significance, the scatter plot relationships and the magnitude of the BIC index. Based on this, the inventors set up two nodes of spline regression based on extensive research, namely 1 ng/mL and 9 ng/mL.

Table 5. Results of spline regression fitting for the relationship between Roche and Beckmann

| Parameters | Standard Error | t value | P value | Lower limit of the 95% CI | Upper limit of the 95% CI |
|---|---|---|---|---|---|
| Intercept | 0.151199 | 0.05 | 0.9565 | -0.28809 | 0.30461 |
| <1 | 0.195303 | 5.37 | <.0001 | 0.665236 | 1.430824 |
| 1-9 | 0.210946 | 2.31 | 0.0217 | 0.073606 | 0.900514 |
| >9 | 0.072011 | -7.81 | <.0001 | -0.70344 | -0.42116 |

According to the results in Table 5, the corresponding relationship between Beckman AMH and Roche AMH was established as follows:

Beckman AMH = a3 + b3*Roche AMH + c3*g1 (Roche AMH-1) + d3*g2 (Roche AMH-9),

in the formula, if Roche AMH was≤ 1 ng/mL, then g1=0, g2=0, and the formula was:

$$\text{Beckman AMH} = a3 + b3 * \text{Roche AMH};$$

if Roche AMH was >1 ng/mL and ≤9 ng/mL, then g1=1, g2=0, and the formula was:

$$\text{Beckman AMH} = a3 + b3 * \text{Roche AMH} + c3 * (\text{Roche AMH-1});$$

if Roche AMH was >9 ng/mL, then g1=1, g2=1, and the formula was:

Beckman AMH = a3 + b3*Roche AMH + c3*(Roche AMH-1) + d3*(Roche AMH-9);

wherein a3 was any value selected from a range of -0.28809 to 0.30461; b3 was any value selected from a range of 0.665236 to 1.430824; c3 was any value selected from a range of 0.073606 to 0.900514; d3 was any value selected from a range of -0.70344 to -0.42116.

Table 6 showed the comparison of the fitting effects of spline regression and Passing-Bablok regression. It can be seen that the RMSE, AIC and AICc of spline regression were lower than those of Passing-Bablok regression, while $R^2$ and corrected $R^2$ were both higher than those of Passing-Bablok regression. Table 7 showed the spline regression estimates for converting AMH results from the Roche method to the Beckmann method.

Table 6. Comparison of spline regression and Passing-Bablok regression during conversion from Roche AMH to Beckmann AMH

| | Spline regression | Passing-Bablok regression |
|---|---|---|
| **RMSE** | 0.858 | 0.943 |
| ***$R^2$*** | 0.977 | 0.972 |
| **Corrected *$R^2$*** | 0.977 | 0.972 |
| **AIC** | 207.138 | 260.130 |
| **AICc** | 207.349 | 260.214 |

Table 7. Converting AMH assays from Roche AMH to Beckman AMH using spline regression

| | Estimated value | P-Value |
|---|---|---|
| Intercept | 0.00826 | 0.9565 |
| <1 | 1.04803 | <.0001 |

(continued)

|  | Estimated value | P-Value |
|---|---|---|
| 1-9 | 0.48706 | 0.0217 |
| >9 | -0.5623 | <.0001 |

Further, a method for converting Beckman AMH(x) to Roche AMH(y) was described based on the regression results.

Table 8. Results of spline regression fitting for the relationship between Roche and Beckman

| Parameters | Standard Error | t value | P value | Lower limit of the 95% CI | Upper limit of the 95% CI |
|---|---|---|---|---|---|
| Intercept | 0.215021 | -0.01 | 0.9881 | -0.42466 | 0.41822 |
| <0.5 | 0.583073 | 1.77 | 0.0781 | -0.11202 | 2.17363 |
| 0.5-1 | 0.810857 | -0.46 | 0.6468 | -1.96122 | 1.21734 |
| >1 | 0.295846 | 0.29 | 0.7724 | -0.49419 | 0.66553 |

According to the results in Table 8, the corresponding relationship between Roche AMH (y) and Beckman AMH (x) was established as follows:

Roche AMH = a4 + b4*Beckmann AMH + c4*g1 (Beckmann AMH-0.5) + d4*g2 (Beckmann AMH-1);

in the formula, if the Beckman AMH value was ≤ 0.5 ng/mL, then g1=0, g2=0, and the formula was:

$$\text{Roche AMH} = a4 + b4*\text{Beckmann AMH};$$

if the Beckman AMH value was >0.5 ng/mL and ≤1 ng/mL, then g1=1, g2=0, and the formula was:

$$\text{Roche AMH} = a4 + b4*\text{Beckmann AMH} + c4*(\text{Beckmann AMH} - 0.5);$$

if the Beckman AMH value was > 1 ng/mL, then g1 = 1, g2 = 1, and the formula was:

Roche AMH = a4 + b4*Beckmann AMH + c4*(Beckmann AMH-0.5) + d4*(Beckmann AMH-1);

a4 was any value selected from a range of -0.42466 to 0.41822; b4 was any value selected from a range of -0.11202 to 2.17363; c4 was any value selected from a range of -1.96122 to 1.21734; d4 is any value selected from a range of -0.49419 to 0.66553.

Taking the conversion of Beckman AMH to Roche AMH as an example, the AMH value measured by Beckman was used as the independent variable x, and the AMH value measured by Roche was used as the dependent variable y. According to the relationship between the independent variable and the dependent variable in the data of the example, a linear spline regression was further employed, that is, a linear regression with smooth connections at the nodes was constructed. In Example 3 (2), the selection of nodes was mainly based on the principle of "the less variation, the better the formula". Based on this, the inventors set two spline regression nodes, namely 0.5 ng/mL and 1 ng/mL, based on extensive research.

Further, the method of converting Beckman AMH(x) to Kangrun AMH(y) was described based on the regression results.

Table 9. Results of spline regression fitting for the relationship between Kangrun and Beckman

| Parameters | Standard Error | t value | P value | Lower limit of the 95% CI | Upper limit of the 95% CI |
|---|---|---|---|---|---|
| Intercept | 0.128467 | 0.08 | 0.9331 | -0.24101 | 0.26258 |
| <=1 | 0.177026 | 6.06 | <.0001 | 0.72643 | 1.42037 |
| >1-4 | 0.210290 | -1.75 | 0.0805 | -0.78107 | 0.04326 |

(continued)

| Parameters | Standard Error | t value | P value | Lower limit of the 95% CI | Upper limit of the 95% CI |
|---|---|---|---|---|---|
| >4-13 | 0.072428 | 6.03 | <.0001 | 0.29488 | 0.57880 |
| >13 | 0.062054 | -9.55 | <.0001 | -0.71455 | -0.47130 |

According to the results in Table 9, the corresponding relationship between Beckman AMH(x) and Kangrun chemiluminescent AMH(y) was established as follows:

Kangrun chemiluminescent AMH = a5+b5*Beckman AMH+c5*g1(Beckman AMH-1)+d5*g2(Beckman AMH-4)+e5*g3 (Beckman AMH-13);

in the formula, if the Beckman AMH value was ≤ 1 ng/mL, then g1=0, g2=0, g3=0, and the formula was:

$$\text{Kangrun chemiluminescent AMH} = a5 + b5 * \text{Beckmann AMH};$$

if the Beckman AMH value was >1 ng/mL and ≤4 ng/mL, then g1=1, g2=0, g3=0, and the formula was:

Kangrun chemiluminescent AMH = a5+b5*Beckman AMH+c5*(Beckman AMH-1);

if the Beckman AMH value was >4 ng/mL and ≤13 ng/mL, then g1=1, g2=1, g3=0, and the formula was:

Kangrun chemiluminescent AMH = a5+b5*Beckman AMH+c5*(Beckman AMH-1)+d5*(Beckman AMH-4);

if the Beckman AMH value was >13 ng/mL, then g1=1, g2=1, g3=1, and the formula was:

Kangrun chemiluminescent AMH = a5 + b5*Beckman AMH + c5*(Beckman AMH-1) + d5*(Beckman AMH-4) + e5* (Beckman AMH-13).

Among them, a5 was any value selected from -0.24101 ~ 0.26258; b5 was any value selected from a range of 0.72643 to 1.42037; c5 was any value selected from a range of -0.78107 to 0.04326; d5 was any value selected from a range of 0.29488 to 0.57880; e5 was any value selected from a range of -0.71455 to -0.47130.

Taking Beckman AMH versus Kangrun chemiluminescent AMH as an example, the AMH value measured by Beckman was used as the independent variable x, and the AMH value measured by Kangrun chemiluminescence was used as the dependent variable y. According to the relationship between the independent variable and the dependent variable in the data of the example, a linear spline regression was further employed, that is, a linear regression with smooth connections at the nodes was constructed. In Example 3 (2), the selection of nodes was mainly based on the principle of "the less variation, the better the formula". Based on this, the inventors set three spline regression nodes, namely 1 ng/mL, 4 ng/mL and 13 ng/mL, based on extensive research.

Further, based on the regression results, the method of converting Kangrun chemiluminescent AMH(x) to Beckmann AMH(y) was described.

Table 10. Results of spline regression fitting for the relationship between Kangrun Chemiluminescence and Beckman

| Parameters | Standard Error | t value | P value | Lower limit of the 95% CI | Upper limit of the 95% CI |
|---|---|---|---|---|---|
| Intercept | 0.254434 | 0.19 | 0.8509 | -0.45082 | 0.54656 |
| <0.5 | 0.554841 | 1.32 | 0.1868 | -0.35329 | 1.82169 |
| 0.5-13 | 0.560592 | 0.82 | 0.4112 | -0.63742 | 1.56010 |
| >13 | 0.075319 | -4.98 | <.0001 | -0.52283 | -0.22757 |

According to the results in Table 10, the corresponding relationship between Beckman AMH and Kangrun chemiluminescent AMH was established as follows:

Beckman AMH = a6 + b6*Kangrun chemiluminescent AMH + c6*g1 (Kangrun chemiluminescent AMH-0.5) + d6*g2 (Kangrun chemiluminescent AMH-13);

in the formula, if the Kangrun chemiluminescent AMH value was ≤ 0.5, then g1 = 0, g2 = 0, and the formula was:

$$\text{Beckman AMH} = a6 + b6*\text{Kangrun chemiluminescent AMH};$$

if the Kangrun chemiluminescent AMH value was >0.5 and ≤13, then g1=1, g2=0, and the formula was:

Beckman AMH = a6+b6*Kangrun chemiluminescent AMH+c6*(Kangrun AMH-0.5);

if the Kangrun chemiluminescent AMH value was >13, then g1 = 1, g2 = 1, and the formula was:

Beckman AMH = a6 + b6*Kangrun chemiluminescent AMH + c6* (Kangrun chemiluminescent AMH-0.5) + d6* (Kangrun AMH-13).

Among them, a6 was any value selected from a range of -0.45082 to 0.54656; b6 was any value selected from a range of -0.35329 to 1.82169; c6 was any value selected from a range of -0.63742 to 1.56010; d6 was any value selected from a range of -0.52283 to -0.22757.

Taking Kangrun chemiluminescent AMH versus Beckman AMH as an example, the AMH value measured by Kangrun Chemiluminescence was used as the independent variable x, and the AMH value measured by Beckman was used as the dependent variable y. According to the relationship between the independent variable and the dependent variable in the data of the example, a linear spline regression was further employed, that is, a linear regression with smooth connections at the nodes was constructed. In Example 3 (2), the selection of nodes is mainly based on the principle of "the less variation, the better the formula". Based on this, the inventors set three spline regression nodes, namely 0.5 ng/mL and 13 ng/mL, based on extensive research.

Based on a thorough analysis of Example 3, the formula for spline regression was employed when the intercept does not include 0; and for the conversion between Roche AMH and Kangrun Chemiluminescent AMH, the formula for Passing-Bablok regression was employed when the intercept includes 0.

Table 11. Four conversions using spline regression when the 95% CI of the intercept using Passing-Bablok regression does not include 0

| | Original data | | | Converted or normalized values | | | | | | | |
| | | | | Conversion of Kangrun Chemiluminescence to Beckman | | Conversion of Roche to Beckman | | Conversion of Beckman to Roche | | Conversion of Beckman to Kangrun Chemiluminescence | |
| Samples | Beckman AMH | Roche AMH | Kangrun chemiluminescent AMH | Spline regression | Passing-Bablok regression | Spline Regression | Passing-Bablok regression | Spline Regression | Passing-Bablok regression | Spline Regression | Passing-Bablok regression |
|---|---|---|---|---|---|---|---|---|---|---|---|
| 1 | 0.08 | 0.08 | 0.06 | 0.09 | -0.03 | 0.10 | 0.00 | 0.08 | 0.15 | 0.10 | 0.16 |
| 2 | 0.08 | 0.05 | 0.08 | 0.11 | -0.01 | 0.06 | -0.05 | 0.08 | 0.15 | 0.10 | 0.16 |
| 3 | 0.09 | 0.10 | 0.08 | 0.11 | -0.01 | 0.12 | 0.02 | 0.09 | 0.16 | 0.11 | 0.17 |
| 4 | 0.09 | 0.11 | 0.10 | 0.12 | 0.01 | 0.12 | 0.02 | 0.09 | 0.16 | 0.11 | 0.17 |
| 5 | 0.12 | 0.11 | 0.10 | 0.12 | 0.01 | 0.13 | 0.04 | 0.12 | 0.18 | 0.14 | 0.20 |
| 6 | 0.45 | 0.51 | 0.50 | 0.42 | 0.47 | 0.54 | 0.55 | 0.46 | 0.43 | 0.49 | 0.49 |
| 7 | 0.47 | 0.44 | 0.50 | 0.42 | 0.47 | 0.47 | 0.47 | 0.48 | 0.45 | 0.52 | 0.50 |
| 8 | 0.39 | 0.44 | 0.51 | 0.43 | 0.48 | 0.47 | 0.47 | 0.40 | 0.38 | 0.43 | 0.43 |

(continued)

| Samples | Original data | | | Converted or normalized values | | | | | | | |
| | | | | Conversion of Kangrun Chemiluminescence to Beckman | | Conversion of Roche to Beckman | | Conversion of Beckman to Roche | | Conversion of Beckman to Kangrun Chemiluminescence | |
| | Beckman AMH | Roche AMH | Kangrun chemiluminescent AMH | Spline regression | Passing-Bablok regression | Spline Regression | Passing-Bablok regression | Spline Regression | Passing-Bablok regression | Spline Regression | Passing-Bablok regression |
|---|---|---|---|---|---|---|---|---|---|---|---|
| 9 | 0.42 | 0.40 | 0.52 | 0.44 | 0.49 | 0.42 | 0.41 | 0.43 | 0.41 | 0.46 | 0.46 |
| 10 | 0.30 | 0.47 | 0.53 | 0.45 | 0.50 | 0.50 | 0.50 | 0.31 | 0.32 | 0.33 | 0.35 |
| 11 | 1.29 | 0.98 | 1.15 | 1.19 | 1.21 | 1.03 | 1.17 | 1.06 | 1.07 | 1.29 | 1.22 |
| 12 | 1.36 | 1.03 | 1.15 | 1.19 | 1.21 | 1.10 | 1.24 | 1.11 | 1.12 | 1.34 | 1.28 |
| 13 | 1.21 | 0.50 | 1.16 | 1.20 | 1.22 | 0.53 | 0.54 | 1.00 | 1.01 | 1.23 | 1.15 |
| 14 | 1.28 | 0.88 | 1.17 | 1.22 | 1.23 | 0.93 | 1.04 | 1.05 | 1.06 | 1.28 | 1.21 |
| 15 | 1.30 | 1.20 | 1.17 | 1.22 | 1.23 | 1.36 | 1.46 | 1.07 | 1.08 | 1.30 | 1.23 |
| 16 | 2.08 | 1.75 | 2.00 | 2.21 | 2.17 | 2.21 | 2.18 | 1.65 | 1.67 | 1.85 | 1.92 |
| 17 | 2.20 | 1.72 | 2.02 | 2.23 | 2.20 | 2.16 | 2.14 | 1.74 | 1.77 | 1.93 | 2.02 |
| 18 | 2.05 | 1.71 | 2.04 | 2.26 | 2.22 | 2.15 | 2.13 | 1.62 | 1.65 | 1.82 | 1.89 |
| 19 | 2.03 | 1.74 | 2.06 | 2.28 | 2.24 | 2.19 | 2.17 | 1.61 | 1.64 | 1.81 | 1.87 |
| 20 | 1.86 | 2.09 | 2.09 | 2.32 | 2.28 | 2.73 | 2.62 | 1.48 | 1.51 | 1.69 | 1.72 |
| 21 | 3.62 | 2.58 | 3.24 | 3.69 | 3.59 | 3.48 | 3.27 | 2.79 | 2.85 | 2.93 | 3.27 |
| 22 | 3.64 | 2.32 | 3.26 | 3.71 | 3.61 | 3.08 | 2.93 | 2.81 | 2.86 | 2.94 | 3.29 |
| 23 | 4.08 | 2.76 | 3.27 | 3.73 | 3.62 | 3.76 | 3.50 | 3.13 | 3.20 | 3.29 | 3.67 |
| 24 | 3.90 | 2.49 | 3.36 | 3.83 | 3.72 | 3.34 | 3.15 | 3.00 | 3.06 | 3.13 | 3.52 |
| 25 | 4.08 | 2.82 | 3.42 | 3.91 | 3.79 | 3.85 | 3.58 | 3.13 | 3.20 | 3.29 | 3.67 |
| 26 | 23.70 | 18.50 | 18.12 | 19.56 | 20.53 | 22.58 | 24.13 | 17.74 | 18.17 | 15.16 | 20.90 |
| 27 | 21.38 | 14.30 | 18.62 | 19.97 | 21.10 | 18.49 | 18.62 | 16.02 | 16.40 | 13.16 | 18.87 |
| 28 | 21.97 | 17.10 | 20.53 | 21.54 | 23.27 | 21.22 | 22.29 | 16.45 | 16.85 | 14.49 | 19.39 |
| 29 | 18.58 | 18.90 | 21.73 | 22.52 | 24.64 | 22.97 | 24.65 | 13.93 | 14.26 | 15.35 | 16.40 |
| 30 | 22.16 | 20.50 | 23.11 | 23.65 | 26.21 | 24.52 | 26.75 | 16.60 | 17.00 | 16.11 | 19.56 |

Recently, AMH has been regarded as the power to regulate ovarian reserve and PCOS. However, the significant discrepancies among different AMH detection methods have evidently hindered the application of AI tools related to AMH. More importantly, the significant discrepancies in detection methods had also led to multiple AMH detections among different testing centers or hospitals. The inventors of this application first proposed the idea of using Passing-Bablok + spline regression to convert AMH concentration from one detection method to another, and through in-depth research, confirmed the most suitable methods and systems for standardizing or converting data from the three detection methods, facilitating practical application.

The idea behind the Passing-Bablok regression + spline regression in the present application was to first apply Passing-Bablok regression to detect the presence of systematic differences, that is, to examine whether the 95% CI of the intercept in the Passing-Bablok regression result included 0. If there were no systematic differences, i.e., the intercept included the value 0, the Passing-Bablok regression method could be used for AMH conversion between the two platforms. If there were systematic differences, spline regression would be used. In the spline regression process, the AIC method could be applied to the selecting the number of nodes. The smaller the AIC, the smaller the variation. And the specific values of these nodes have been fully studied and adjusted according to the calculated conversion values and their original values in the Examples, ultimately confirming the most suitable checkpoint data.

Three AMH detection methods were used in the study of this Example. The applicant found that certain individual samples yielded significantly lower values using the Roche AMH detection method compared to the other two platforms. Interference caused by biotin was considered the key reason for the occasional occurrence of low values when using the Roche AMH detection method. For the AMH kit, Roche chosed to use a biotinylated AMH secondary antibody, which introduced the potential for biotin interference into the design, unlike the other two companies. A small number of patients visiting the ART clinic often taken health supplements or medicines comprising biotin, such as vitamin B7, vitamin H,coenzyme R, etc. For individuals taking supplements comprising biotin, biotin would be present in their blood and bound to the avermectin magnetic beads, thereby reducing the available binding sites on the avermectin magnetic beads and reducing the AMH measurement value of the Roche platform.

In this example, the inventors found that, first, there was no systematic difference between the Roche method and the Kangrun chemiluminescent method, and the 95% CI of the intercept covers 0, and only a proportional difference existed. The three commercially available AMH assays performed well in terms of accuracy and precision. In our study, the conversion of three detection methods allowed one AMH detection method to be conveted to another without the need for additional testing. Thus, it might facilitate the application of AMH-related tools and reduced costs for patients undergoing assisted reproductive technology when visiting different clinics.

### Example 4

For $E_2$ detection, serum samples were donated by patients visiting the Reproductive Center of Peking University Third Hospital. The subjects provided written informed consent to donate their remaining serum for research after the original blood test. This experiment has received ethical certification from the Institutional Review Board of Peking University Third Hospital, China.

### $E_2$ detection

The same serum samples were subjected to freeze-thaw cycles and detected on two different manufacturers' SIEMENS Immulite 2000 automated immunoassay systems (Siemens Healthineers Diagnostics (Shanghai) Co., Ltd., Shanghai, China) and KANGRUN Keaser 6600 automated immunoassay systems (KANGRUN Inc., Guangzhou, Guangdong Province, China), respectively. The $E_2$ assays on both automated systems were performed once according to the manufacturer's recommendations. All $E_2$ analysis results were reported in pmol/L. Quality control for the $E_2$ assay was provided by Bio-RAD Laboratories (Lyphochek Immunoassay Plus Control, Trilevel, Cat. No. 370, Batch No. 40370). The coefficient of variation of the trilevel control was less than 10%.

### 4.1 Regression results of Passing-Bablok

Passing-Bablok regression was used to construct the linear relationship between Siemens $E_2$ detection value and Kangrun $E_2$ detection value. Figure 2 showed the fitting results. In Figure 2, the dashed black line represented the scenario where the two measurements are perfectly consistent, while the black straight fitting line represented the Passing-Bablok regression result. As can be seen from the figure, the linear relationship between the two measurements showed a certain deviation from the dashed line, and this difference required statistical testing.

Table 12 showed the analysis results of Passing-Bablok regression. The results showed that the 95% confidence interval of the intercept estimate did not include 0, indicating that the intercept was statistically different from 0; the 95% confidence interval of the slope estimate did not include 1, indicating that the slope was statistically different from 1. The intercept term mainly reflected the systematic difference. Since the 95% confidence interval did not include 0, it suggested that there was a systematic difference between the two measurement results. The slope mainly reflected the difference in proportion. Since the 95% confidence interval did not include 1, it suggested that there might be a difference in proportion between the two measurement results.

Table 12. Regression results of Passing-Bablok

| Parameters | Estimate | Low CL | High CL |
|---|---|---|---|
| Intercept | 24.4697 | 14.9212 | 35.5684 |
| Slope | 0.94238 | 0.90687 | 0.99069 |

Fig. 3 shows the Bland-Altman plot. Bland-Altman plots serve as a complement to Passing-Bablok regression, graphically exploring the consistency between two measurements. In Fig. 3, the horizontal axis represented the mean values of the $E_2$ detection results from Kangrun and Siemens, while the vertical axis represented the differences between the $E_2$ detection values from Kangrun and Siemens. The black solid line in the Figure represented the reference line, i.e., the 0 value; the solid line and dashed line in the middle represent the mean difference and its 95% confidence interval, respectively; the upper and lower lines of the gray box (i.e., the gray area in the Figure) represented the 95% distribution range of the difference (mean $\pm 1.96 \times$ standard deviation), which was also referred to as the consistency boundaries. In theory, if the distribution of the differences followed a normal distribution, 95% of the differences should be within the consistency boundaries.

The higher the consistency between the two measurements, the closer the middle solid line representing the mean differences was to the reference line (the solid gray line representing a mean differences of 0). The consistency of the two methods to be evaluated was evaluated based on the number of data points outside the 95% consistency boundaries and the maximum difference within the consistency boundaries, along with the clinical acceptability. In this Example, a significant number of observations exceeded the consistency boundaries, predominantly showing negative values, indicating that Siemens had significant discrepancies in many detection values compared to Kangrun.

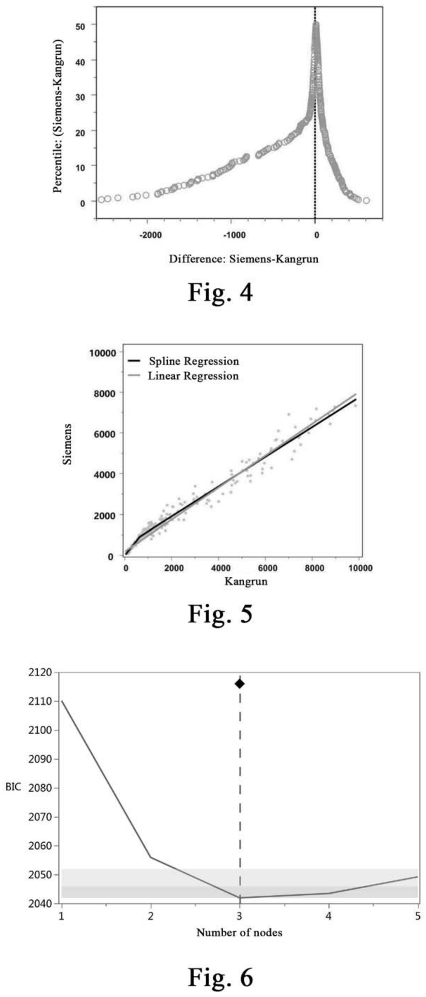

Fig. 4 showed a ridge plot of Siemens $E_2$ detection values versus Kangrun $E_2$ detection values. The horizontal axis of the ridge plot represented the difference between Siemens $E_2$ detection values and Kangrun $E_2$ detection values, while the vertical axis represented the transformation of the cumulative distribution plot, that is, when the cumulative distribution probability was less than 50%, it was the cumulative distribution probability, and when it exceeded 50%, it was 1 minus the cumulative distribution probability. If the two results were consistent, they should be symmetrically distributed with 0 as the center. As can be seen from Fig. 4, "Siemens $E_2$ detection value - Kangrun $E_2$ detection value" is obviously left-skewed, indicating that the difference of "Siemens $E_2$ detection value - Kangrun $E_2$ detection value" was mostly less than 0, and the left tail was longer, suggesting that the difference of "Siemens $E_2$ detection value - Kangrun $E_2$ detection value" was particularly low in some cases. Combined with the Bland-Altman graph in Fig. 3, it can be seen that "Siemens $E_2$ detection value - Kangrun $E_2$ detection value" has more low values.

**4.2 Results of Spline regression**

**(1) Firstly, determined the number of nodes and their values**

The determination of the number of nodes primarily combined clinical and statistical indicators, with statistical indicators mainly included BIC and AIC and other indicators. The lower the BIC and AIC values, the better the model. Finally, based on the statistical indicators of different numbers of nodes and taking actual clinical situations into account, the model with 2 nodes was ultimately selected. The two nodes were 660pmol/L and 2030pmol/L, respectively.

**(2) Fitting the model based on the number of nodes**

The statistical analysis results for the fitted linear spline regression model with 2 knots were shown in Table 13.

Table 13. Spline regression fitting results of the relationship between Kangrun and Siemens

| Parameters | Standard Error | t value | P value | Lower limit of the 95% CI | Upper limit of the 95% CI |
|---|---|---|---|---|---|
| Intercept | 21.97882 | -2.27 | 0.0235 | -93.069 | -6.91207 |
| <=660 | 0.06605 | 21.81 | <.0001 | 1.310892 | 1.569808 |
| >660-2030 | 0.09502 | -7.47 | <.0001 | -0.8957 | -0.52322 |
| >2030 | 0.04346 | 0.00 | 0.9975 | -0.08505 | 0.085318 |

According to the results in Table 13, the corresponding relationship between Siemens $E_2$ detection value (y) and Kangrun $E_2$ detection value (x) was established as follows:

Siemens $E_2$ detection value = a8+b8*Kangrun $E_2$ detection value+c8*g1 (Kangrun $E_2$ detection value-660)+d8*g2 (Kangrun $E_2$ detection value-2030);

in the formula,

if Kangrun $E_2$ detection value was $\leq$ 660pmol/L, then g1=0, g2=0, and the formula is:

$$\text{Siemens } E_2 \text{ detection value} = a8+b8* \text{ Kangrun } E_2 \text{ detection value;}$$

if the Kangrun $E_2$ detection value was >660pmol/L and $\leq$2030pmol/L, then g1=1, g2=0, and the formula was:

Siemens $E_2$ detection value = a8+b8*Kangrun $E_2$ detection value + c8*(Kangrun $E_2$ detection value - 660);

if the Kangrun $E_2$ detection value was >2030pmol/L, then g1=1, g2=1, and the formula was:

Siemens $E_2$ detection value = a8+b8*Kangrun $E_2$ detection value+c8*(Kangrun $E_2$ detection value-660)+d8*(Kangrun $E_2$ detection value-2030);

among them, a8 was any value selected from a range of -93.069 to -6.91207; b8 was any value selected from a range of 1.310892 to 1.569808; c8 was any value selected from a range of -0.8957 to -0.52322; d8 was any value selected from a range of -0.08505 to 0.085318.

Taking Kangrun's $E_2$ detection value versus Siemens' $E_2$ detection value as an example, Kangrun's $E_2$ detection value was used as the independent variable x, and Siemens' $E_2$ detection value was used as the dependent variable y. According to the relationship between the independent variable and the dependent variable in the data of the example, a linear spline regression was further employed, that is, a linear regression with smooth connections at the nodes was constructed. In this Example 4, the selection of nodes was based on the principle of " the less variation, the better the formula". Based on this, the inventors set two spline regression nodes, namely 660 pmol/L and 2030 pmol/L, based on extensive research.

**(3) Evaluation of fitting model**

Table 14 showed the comparison of the fitting effects between spline regression and Passing-Bablok regression. It can be seen that the root mean square error, AIC, and AICc of spline regression are lower than those of Passing-Bablok regression, while $R^2$ and adjusted $R^2$ are higher than those of linear regression, indicating that the fitting effect of spline regression is better.

Table 14. Comparison of fitting indicators between spline regression and linear regression

| | Passing-Bablok regression | Spline Regression |
|---|---|---|
| Root mean square error | 261.51380 | 220.23999 |
| $R^2$ | 0.9760 | 0.9830 |
| Adjust $R^2$ | 0.9759 | 0.9829 |
| AIC | 4772.25372 | 4639.22771 |
| AICc | 4772.31542 | 4639.38275 |

**(4) Spline regression prediction**

Fig. 5 showed a comparison of predictions using Passing-Bablok regression and spline regression. It can be seen that Passing-Bablok regression demonstrates significant bias in prediction within the low-value range (Kangrun $E_2$ detection value was less than 1000), whereas spline regression provides more reasonable predicted values.

**Example 5**

For the two tubes of frozen serum of each sample obtained in Example 1, one tube was detected using the Kangrun chemiluminescent method after thawing, and the other tube was detected using the Kangrun enzyme immunoassay (Anshlab) AMH detection method.

Generally speaking, if the AMH data of Kangrun chemiluminescence detection and the AMH detection data of Kangrun enzyme immunoassay (Anshlab) AMH assay are consistent with the overall relationship, Passing-Bablok regression can be considered for pairwise data conversion. Passing-Bablok regression used a non-parametric approach to fit the parameters a and b in the linear equation y=a+bx. If the 95% confidence interval (CI) for the intercept a did not comprise 0 or the 95% CI for the slope b did not comprise 1, it indicated the presence of systematic or proportional differences between the two AMH detection results.

If the detection results of the two platform methods did not satisfy the overall relationship, spline regression can be considered to find the conversion between the detection data of the two platforms. Spline regression is a piecewise polynomial function that is smoothly connected at the nodes. It can be a linear relationship (straight lines connected at the nodes), a quadratic relationship, or a cubic relationship. In spline regression, based on the relationship between the independent variable and the dependent variable, linear spline regression can be used, attempting to construct a linear regression with smooth connections at the nodes.

For the convenience of practical application, linear spline regression was employed to further determine the number of nodes.

The number of nodes was determined based on the BIC indicator. The lower the BIC, the better the fitting effect of the model. We designated 1 to 5 nodes separately, and the results indicated that the model fitted best with 3 nodes, as shown in Figure 6.

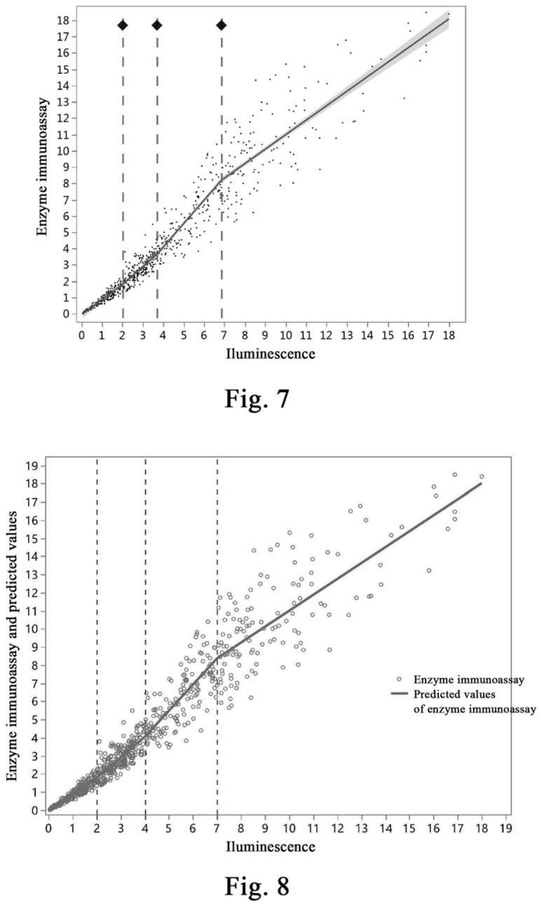

The three nodes were 2 ng/mL, 3.7 ng/mL and 6.9 ng/mL, respectively. Considering the convenience of practical application, we rounded them off, setting the three nodes at 2 ng/mL, 4 ng/mL and 7 ng/mL, dividing the data into 4 segments as shown in Fig. 7.

The following was a linear spline regression fitting:

the fitting effect of 3-node linear spline regression was as follows:

$R^2$ and corrected $R^2$ were 0.9326 and 0.9322, respectively, and AIC and AICc were 713.816 and 713.936, respectively.

Parameter estimates

| Parameters | Standard error | t value | P value | Lower limit of the 95% CI | Upper limit of the 95% CI |
|---|---|---|---|---|---|
| Intercept | 0.130482 | -0.42 | 0.6777 | -0.31000 | 0.20149 |
| Slope 1 | 0.092349 | 9.96 | <.0001 | 0.73863 | 1.10064 |
| Slope 2 | 0.152911 | 1.38 | 0.1695 | -0.08941 | 0.51000 |
| Slope 3 | 0.118522 | 2.65 | 0.0083 | 0.08121 | 0.54582 |
| Slope 4 | 0.070805 | -8.00 | <.0001 | -0.70493 | -0.42738 |

* Slope 1 represented the slope of the first segment of data, slope 2 represented the change in slope of the second segment of data compared to the first segment of data; slope 3 represented the change in slope of the 2nd segment of data compared to the 3rd segment of data; slope 4 represented the change in slope of the 3rd segment of data compared to the 4th segment of data. The formula summarized from this was as follows:

the formula for converting Kangrun chemiluminescent AMH (X) to Kangrun chemiluminescent AMH (Y) is:

Kangrun enzyme immunoassay (Anshlab) AMH = a7 + b7*Kangrun chemiluminescent AMH + c7*g1*(Kangrun chemiluminescent AMH-2) + d7*g2* (Kangrun chemiluminescent AMH-4) + e7*g3*(Kangrun chemiluminescent AMH-7);

wherein, a7 was any value selected from a range of -0.31000 to 0.20149, b7 was any value selected from a range of 0.73863 to 1.10064, c7 was any value selected from a range of -0.08941 to 0.51000, d7 was any value selected from a range of 0.08121 to 0.54582, and e7 was any value selected from a range of -0.70493 to -0.42738; further,

if Kangrun chemiluminescent AMH was ≤ 2 ng/mL, then g1 = 0, and the formula was:

Kangrun enzyme immunoassay (Anshlab) AMH = a7 + b7*Kangrun chemiluminescent AMH;

if Kangrun chemiluminescent AMH was >2ng/mL and ≤4ng/mL, then g1=1, g2=0, and the formula was:

Kangrun enzyme immunoassay (Anshlab) AMH = a7 + b7*Kangrun chemiluminescent AMH + c7*g1 (Kangrun chemiluminescent AMH-2);

if Kangrun chemiluminescent AMH was >4 ng/mL and ≤7 ng/mL, then g1=1, g2=1, and the formula was:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4);

if Kangrun chemiluminescent AMH was >7 ng/mL, then g1 = 1, g2 = 1, g3 = 1, and the formula was:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4) + e7*g3 (Kangrun chemiluminescent AMH-7).

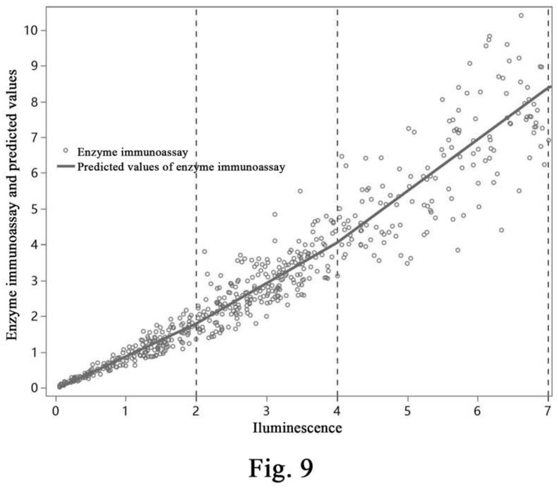

The prediction effect was shown in Fig. 8. Wherein, the prediction effect of the first three data segments with values below 7 ng/mL was as shown in Figure 9.

This application uses both Passing-Bablok regression and spline regression to construct the conversion relationship between Kangrun and Siemens. The assumption of Passing-Bablok regression is that both test results are random, primarily to assess the consistency of the two test results, comparing the difference between the intercept and 0 and the difference between the slope and 1, respectively. If both tests show no statistical significance, it can be concluded that the two test results are consistent and can be interchanged; otherwise, the two test results cannot be considered consistent, and the results cannot be interchanged, and they need to be converted by other corresponding formulas. If there is an obvious linear relationship between the two detection results, conversion can be achieved through Passing-Bablok regression; if a linear relationship is not present, spline regression may be considered. Both Passing-Bablok regression and spline regression can give clear conversion formulas.

Although the embodiments of the present application are described above, the present application is not limited to the aforementioned specific embodiments and application fields. The aforementioned specific embodiments are merely illustrative and instructive, but not restrictive. Those ordinary skilled in the art may also make many forms under the inspiration of this specification and without departing from the protection scope of the claims of the present application, which are all within the protection scope of the present application.

## Claims

1. A method for converting hormone data detected by different detection methods, comprising:

   obtaining a first detection value for the hormone from a first platform;
   obtaining a second detection value for the hormone from a second platform;
   using Passing-Bablok regression to fit the first and the second detection values;
   determining whether there is a systematic difference between the first and the second detection values based on the Passing-Bablok regression fitting results;
   converting the first detection value or the second detection value based on the fitting formula of Passing-Bablok regression, if there is no systematic difference between the first and the second detection values;
   fitting the first and the second detection values based on spline regression, and converting the first detection value or the second detection value based on the fitting formula of the spline regression, if there is a systematic difference between the first and the second detection values.

2. The method according to claim 1, wherein the determination of whether there is a systematic difference between the first and the second detection values based on the Passing-Bablok regression fitting results is to judge whether there is a systematic difference between the first and second detection values by calculating the 95% confidence interval of the intercept from the Passing-Bablok regression results.

3. The method according to claim 2, wherein

   the 95% confidence interval of the intercept comprises 0, indicating no systematic difference between the first and the second detection values;
   the 95% confidence interval of the intercept does not comprise 0, indicating the presence of systematic difference between the first and the second detection values.

4. The method according to any one of claims 1 to 3, wherein
when fitting the first and the second detection values based on the spline regression, intrinsic nodes and statistical indicators are utilized to confirm the nodes of the spline regression.

5. The method according to claim 4, wherein the statistical indicator is an AIC (Akaike Information Criterion) or a BIC (Bayesian Information Criterion) indicator.

6. The method according to any one of claims 1 to 5, wherein the hormone is a sex hormone, preferably the sex hormone is selected from hypothalamic-pituitary-gonadal axis hormone, comprising hypothalamic hormone, pituitary prolactin (PRL), follicle-stimulating hormone (FSH), luteinizing hormone (LH), anti-Müllerian hormone(AMH), inhibin B, estradiol ($E_2$), testosterone (T), dehydroepiandrosterone sulfate ((DHEA-S), estriol ($E_3$), and progesterone (P); preferably anti-Müllerian hormone (AMH) or estradiol ($E_2$).

7. The method according to claim 6, wherein the sex hormone is anti-Müllerian hormone (AMH), wherein when the first platform is the Roche AMH detection platform and the second platform is the Kangrun chemiluminescent AMH detection platform, Passing-Bablok regression is used to convert the detection results of the Roche AMH detection platform and the Kangrun chemiluminescent AMH detection platform;

preferably, the formula for converting Kangrun chemiluminescent AMH (X) to Roche AMH (Y) is:

$$\text{Roche AMH} = a1 + b1*\text{Kangrun chemiluminescent AMH};$$

wherein a1 is any value selected from a range of -0.0035 to 0.0303; b1 is any value selected from a range of 0.85 to 0.8844;
preferably, the formula for converting Roche AMH (X) to Kangrun chemiluminescent AMH (Y) is:

$$\text{Kangrun chemiluminescent AMH} = a2 + b2*\text{Roche AMH};$$

wherein a2 is any value selected from a range of -0.0356 to 0.00393; b2 is any value selected from a range of 1.1307 to 1.1765.

8. The method according to claim 6, wherein the sex hormone is anti-Müllerian hormone (AMH), wherein when the first platform is the Beckman AMH detection platform and the second platform is the Roche AMH detection platform, spline regression is used to convert the detection results of the Beckman AMH detection platform and the Roche AMH detection platform;

preferably, the formula for converting Roche AMH (X) to Beckman AMH (Y) is:

Beckman AMH = a3 + b3*Roche AMH + c3*g1 (Roche AMH-1) + d3*g2 (Roche AMH-9),

in the formula, if Roche AMH is ≤ 1 ng/mL, then g1 = 0, g2 = 0, and the formula is:

$$\text{Beckman AMH} = a3 + b3*\text{Roche AMH};$$

if Roche AMH is >1 ng/mL and ≤9 ng/mL, then g1=1, g2=0, and the formula is:

$$\text{Beckman AMH} = a3 + b3*\text{Roche AMH} + c3*(\text{Roche AMH-1});$$

if Roche AMH is >9 ng/mL, then g1=1, g2=1, and the formula is:

Beckman AMH = a3 + b3*Roche AMH + c3*(Roche AMH-1) + d3*(Roche AMH-9);

wherein a3 is any value selected from a range of -0.28809 to 0.30461; b3 is any value selected from a range of 0.665236 to 1.430824; c3 is any value selected from a range of 0.073606 to 0.900514; d3 is any value selected from a range of -0.70344 to -0.42116;

preferably, the formula for converting Beckman AMH (X) to Roche AMH (Y) is:

Roche AMH = a4 + b4*Beckmann AMH + c4*g1 (Beckmann AMH-0.5) + d4*g2 (Beckmann AMH-1),

in the formula, if the Beckman AMH value is ≤ 0.5 ng/mL, then g1=0, g2=0, and the formula is:

$$\text{Roche AMH} = a4 + b4*\text{Beckmann AMH};$$

if the Beckman AMH value is >0.5 ng/mL and ≤1 ng/mL, then g1=1, g2=0, and the formula is:

$$\text{Roche AMH} = a4 + b4*\text{Beckmann AMH} + c4*(\text{Beckmann AMH} - 0.5);$$

if the Beckman AMH value is > 1 ng/mL, then g1 = 1, g2 = 1, and the formula is:

Roche AMH = a4+b4*Beckmann AMH + c4*(Beckmann AMH-0.5) + d4*(Beckmann AMH-1);

a4 is any value selected from a range of -0.42466 to 0.41822; b4 is any value selected from a range of -0.11202 to 2.17363; c4 is any value selected from a range of -1.96122 to 1.21734; d4 is any value selected from a range of -0.49419 to 0.66553.

9.  The method according to claim 6, wherein the sex hormone is anti-Müllerian hormone (AMH), wherein the first platform is the Beckman AMH detection platform, and the second platform is the Kangrun chemiluminescent AMH detection platform, and spline regression is used to convert the detection results of the Beckman AMH detection platform and the Kangrun chemiluminescent AMH detection platform;

    preferably, the formula for converting Beckman AMH (X) to Kangrun chemiluminescent AMH (Y) is:

    Kangrun chemiluminescent AMH = a5+b5*Beckman AMH+c5*g1(Beckman AMH-1)+d5*g2(Beckman AMH-4)+e5*g3(Beckman AMH-13);

    in the formula, if the Beckman AMH value is ≤ 1 ng/mL, then g1=0, g2=0, g3=0, and the formula is:

$$\text{Kangrun chemiluminescent AMH} = a5+b5*\text{Beckmann AMH};$$

    if the Beckman AMH value is >1 ng/mL and ≤ 4 ng/mL, then g1=1, g2=0, g3=0, and the formula is:

    Kangrun chemiluminescent AMH = a5+b5*Beckman AMH+c5*(Beckman AMH-1);

    if the Beckman AMH value is >4 ng/mL and ≤13 ng/mL, then g1=1, g2=1, g3=0, and the formula is:

    Kangrun chemiluminescent AMH = a5+b5*Beckman AMH+c5*(Beckman AMH-1)+d5*(Beckman AMH-4);

    if the Beckman AMH value is >13 ng/mL, then g1=1, g2=1, g3=1, and the formula is:

    Kangrun chemiluminescent AMH = a5+b5*Beckman AMH+c5*(Beckman AMH-1)+d5*(Beckman AMH-4)+e5*(Beckman AMH-13),

    wherein, a5 is any value selected from -0.24101 ~ 0.26258; b5 is any value selected from a range of 0.72643 to 1.42037; c5 is any value selected from a range of -0.78107 to 0.04326; d5 is any value selected from a range of 0.29488 to 0.57880; e5 is any value selected from a range of -0.71455 to -0.47130;
    preferably, the formula for converting Kangrun chemiluminescent AMH (X) to Beckman AMH (Y) is:

    Beckman AMH = a6 + b6*Kangrun chemiluminescent AMH + c6*g1 (Kangrun chemiluminescent AMH-0.5) + d6*g2 (Kangrun chemiluminescent AMH-13);

in the formula, if the Kangrun chemiluminescent AMH value is ≤ 0.5 ng/mL, then g1=0, g2=0, and the formula is:

$$\text{Beckman AMH} = a6 + b6 * \text{Kangrun chemiluminescent AMH};$$

If the Kangrun chemiluminescent AMH value is >0.5 and ≤13 ng/mL, then g1=1, g2=0, and the formula is:

Beckman AMH = a6 + b6*Kangrun chemiluminescent AMH + c6*(Kangrun chemiluminescent AMH-0.5);

if the Kangrun chemiluminescent AMH value is >13 ng/mL, then g1=1, g2=1, and the formula is:

Beckman AMH = a6 + b6*Kangrun chemiluminescent AMH + c6*(Kangrun chemiluminescent AMH-0.5) + d6* (Kangrun chemiluminescent AMH-13);

wherein, a6 is any value selected from a range of -0.45082 to 0.54656; b6 is any value selected from a range of -0.35329 to 1.82169; c6 is any value selected from -0.63742 ~ 1.56010; d6 is any value selected from a range of -0.52283 to -0.22757.

10. The method according to claim 6, wherein the sex hormone is anti-Müllerian hormone (AMH), wherein the first platform is Kangrun chemiluminescent AMH detection platform, and the second platform is Kangrun enzyme immunoassay (Anshlab) AMH detection platform, and spline regression is used to convert the detection results of Kangrun chemiluminescent AMH detection platform and Kangrun enzyme immunoassay (Anshlab) AMH detection platform;

preferably, the formula for converting Kangrun chemiluminescent AMH (X) to Kangrun chemiluminescent AMH (Y) is:

Kangrun enzyme immunoassay (Anshlab) AMH = a7 + b7*Kangrun chemiluminescent AMH + c7*g1*(Kangrun chemiluminescent AMH-2) + d7*g2*(Kangrun chemiluminescent AMH-4) + e7*g3*(Kangrun chemiluminescent AMH-7);

wherein, a7 is any value selected from a range of -0.31000 to 0.20149, b7 is any value selected from a range of 0.73863 to 1.10064, c7 is any value selected from a range of -0.08941 to 0.51000, d7 is any value selected from a range of 0.08121 to 0.54582, and e7 is any value selected from a range of -0.70493 to -0.42738; further,
if Kangrun chemiluminescent AMH is ≤ 2 ng/mL, then g1 = 0, and the formula is:

Kangrun enzyme immunoassay (Anshlab) AMH = a7 + b7*Kangrun chemiluminescent AMH;

if Kangrun chemiluminescent AMH is >2ng/mL and ≤4ng/mL, then g1=1, g2=0, and the formula is:

Kangrun enzyme immunoassay (Anshlab) AMH = a7 + b7*Kangrun chemiluminescent AMH + c7*g1 (Kangrun chemiluminescent AMH-2);

if Kangrun chemiluminescent AMH is >4 ng/mL and ≤7 ng/mL, then g1=1, g2=1, and the formula is:

Kangrun enzyme immunoassay (Anshlab) AMH = a7 + b7*Kangrun chemiluminescent AMH + c7*g1(Kangrun chemiluminescent AMH-2) + d7*g2 (Kangrun chemiluminescent AMH-4);

If Kangrun chemiluminescent AMH is >7 ng/mL, then g1= 1, g2=1, g3 = 1, and the formula is:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4) + e7*g3 (Kangrun chemiluminescent AMH-7).

11. The method according to claim 7, wherein the sex hormone is estradiol $E_2$, wherein the first platform is the Kangrun estradiol detection platform and the second platform is the Siemens estradiol detection platform, and spline regression is used to convert the detection results of Kangrun estradiol and Siemens estradiol;

Siemens $E_2$ detection value = a8+b8*Kangrun $E_2$ detection value+c8*g1 (Kangrun $E_2$ detection value-660) + d8*g2 (Kangrun $E_2$ detection value-2030);

in the formula,

if Kangrun $E_2$ detection value is $\leq$ 660pmol/L, then g1=0, g2=0, and the formula is:

$$\text{Siemens } E_2 \text{ detection value} = a8+b8* \text{ Kangrun } E_2 \text{ detection value;}$$

if the Kangrun $E_2$ detection value is >660pmol/L and $\leq$ 2030pmol/L, then g1=1, g2=0, and the formula is:

Siemens $E_2$ detection value = a8+b8*Kangrun $E_2$ detection value + c8*(Kangrun $E_2$ detection value-660);

if Kangrun $E_2$ detection value is >2030pmol/L, then g1=1, g2=1, and the formula is:

Siemens $E_2$ detection value = a8+b8*Kangrun $E_2$ detection value+c8*(Kangrun $E_2$ detection value-660)+d8* (Kangrun $E_2$ detection value-2030);

wherein, a7 is any value selected from a range of -93.069 to -6.91207; b7 is any value selected from a range of 1.310892 to 1.569808; c7 is any value selected from a range of -0.8957 to -0.52322; d7 is any value selected from a range of -0.08505 to 0.085318.

12. A system for converting hormone data detected by different detection methods, comprising:

a submodule for acquiring a first detection value for the hormone from a first platform;
a submodule for acquiring a second detection value for the hormone from a second platform;
a submodule for fitting the first and the second detection values using Passing-Bablok regression;
a submodule for determining whether there is a systematic difference between the first and the second detection values based on the Passing-Bablok regression fitting result;
in the above submodule for determining whether there is a systematic difference between the first and the second detection values based on the Passing-Bablok regression fitting result, the following steps are performed:

if there is no systematic difference between the first and the second detection values, the first detection value or the second detection value is converted based on the fitting formula of Passing-Bablok regression;
if there is a systematic difference between the first and the second detection values, the first and the second detection values are fitted based on spline regression, and the first detection value or the second detection value is converted based on the fitting formula of spline regression.

13. The system according to claim 12, wherein the determination of whether there is a systematic difference between the first and the second detection values based on the Passing-Bablok regression fitting results is to judge whether there is a systematic difference between the first and the second detection values by calculating the 95% confidence interval of the intercept of the linear regression.

14. The system according to claim 13, wherein,

the 95% confidence interval of the intercept comprises 0, indicating that there is no systematic difference between the first and the second detection values;
the 95% confidence interval of the intercept doesn't comprise 0, indicating that there is systematic difference between the first and the second detection values.

15. The system according to any one of claims 12 to 14, wherein

when fitting the first and the second detection values based on the spline regression, the nodes of the spline regression are confirmed by using the intrinsic nodes and the statistical indicator.

16. The system according to claim 15, wherein the statistical indicator is an AIC (Akaike Information Criterion) or a BIC (Bayesian Information Criterion) indicator.

17. The system according to any one of claims 12 to 16, wherein the hormone is a sex hormone, and the sex hormone is selected from hypothalamic-pituitary-gonadal axis hormones, including hypothalamic hormones, pituitary prolactin (PRL), follicle-stimulating hormone (FSH), luteinizing hormone (LH), anti-Müllerian hormone (AMH), inhibin B, estradiol ($E_2$), testosterone (T), dehydroepiandrosterone sulfate (DHEA-S), estriol ($E_3$), progesterone (P), preferably anti-Müllerian hormone (AMH) or estradiol ($E_2$).

18. The system according to claim 17, wherein the sex hormone is anti-Müllerian hormone (AMH), and when the first platform is the Roche AMH detection platform and the second platform is the Kangrun chemiluminescent AMH detection platform, Passing-Bablok regression is used to convert the detection results of the Roche AMH detection platform and the Kangrun chemiluminescent AMH detection platform;

preferably, the formula for converting Kangrun chemiluminescent AMH (X) to Roche AMH (Y) is:

$$\text{Roche AMH} = a1 + b1*\text{Kangrun chemiluminescent AMH};$$

wherein a1 is any value selected from a range of -0.0035 to 0.0303; b1 is any value selected from a range of 0.85 to 0.8844;
preferably, the formula for converting Roche AMH (X) to Kangrun chemiluminescent AMH (Y) is:

$$\text{Kangrun chemiluminescent AMH} = a2 + b2*\text{Roche AMH};$$

wherein a2 is any value selected from a range of -0.0356 to 0.00393; b2 is any value selected from a range of 1.1307 to 1.1765.

19. The system according to claim 17, wherein the sex hormone is anti-Müllerian hormone (AMH), and when the first platform is the Beckman AMH detection platform and the second platform is the Roche AMH detection platform, spline regression is used to convert the detection results of the Beckman AMH detection platform and the Roche AMH detection platform;

preferably, the formula for converting Roche AMH (X) to Beckman AMH (Y) is:

Beckman AMH = a3 + b3*Roche AMH + c3*g1 (Roche AMH-1) + d3*g2 (Roche AMH-9),

in the formula, if Roche AMH is $\leq$ 1 ng/mL, then g1 = 0, g2 = 0, and the formula is:

$$\text{Beckman AMH} = a3 + b3*\text{Roche AMH};$$

if Roche AMH is >1 ng/mL and $\leq$9 ng/mL, then g1=1, g2=0, and the formula is:

$$\text{Beckman AMH} = a3 + b3*\text{Roche AMH} + c3*(\text{Roche AMH-1});$$

if Roche AMH is >9 ng/mL, then g1=1, g2=1, and the formula is:

Beckman AMH = a3 + b3*Roche AMH + c3*(Roche AMH-1) + d3*(Roche AMH-9);

wherein a3 is any value selected from a range of -0.28809 to 0.30461; b3 is any value selected from a range of 0.665236 to 1.430824; c3 is any value selected from a range of 0.073606 to 0.900514; d3 is any value selected from a range of -0.70344 to -0.42116;
preferably, the formula for converting Beckman AMH (X) to Roche AMH (Y) is:

Roche AMH = a4 + b4*Beckmann AMH + c4*g1 (Beckmann AMH-0.5) + d4*g2 (Beckmann AMH-1);

in the formula, if the Beckman AMH value is ≤ 0.5 ng/mL, then g1=0, g2=0, and the formula is:

$$\mathrm{Roche\ AMH = a4 + b4*Beckmann\ AMH;}$$

if the Beckman AMH value is >0.5 ng/mL and ≤1 ng/mL, then g1=1, g2=0, and the formula is:

$$\mathrm{Roche\ AMH = a4 + b4*Beckmann\ AMH + c4*(Beckmann\ AMH - 0.5);}$$

if the Beckman AMH value is > 1 ng/mL, then g1 = 1, g2 = 1, and the formula is:

Roche AMH = a4 + b4*Beckmann AMH + c4*(Beckmann AMH-0.5) + d4*(Beckmann AMH-1);

a4 is any value selected from a range of -0.42466 to 0.41822; b4 is any value selected from a range of -0.11202 to 2.17363; c4 is any value selected from a range of -1.96122 to 1.21734; d4 is any value selected from a range of -0.49419 to 0.66553.

20. The system according to claim 17, wherein the sex hormone is anti-Müllerian hormone (AMH), and when the first platform is the Beckman AMH detection platform and the second platform is the Kangrun chemiluminescent AMH detection platform, spline regression is used to convert the detection results of the Beckman AMH detection platform and the Kangrun chemiluminescent AMH detection platform;

preferably, the formula for converting Beckman AMH (X) to Kangrun chemiluminescent AMH (Y) is:

Kangrun chemiluminescent AMH = a5+b5*Beckman AMH+c5*g1(Beckman AMH-1)+d5*g2(Beckman AMH-4)+e5*g3(Beckman AMH-13);

in the formula, if the Beckman AMH value is ≤ 1 ng/mL, then g1=0, g2=0, g3=0, and the formula is:

$$\mathrm{Kangrun\ chemiluminescent\ AMH = a5+b5*Beckmann\ AMH;}$$

if the Beckman AMH value is >1 ng/mL and ≤4 ng/mL, then g1=1, g2=0, g3=0, and the formula is:

Kangrun chemiluminescent AMH = a5+b5*Beckman AMH+c5*(Beckman AMH-1);

if the Beckman AMH value is >4 ng/mL and ≤13 ng/mL, then g1=1, g2=1, g3=0, and the formula is:

Kangrun chemiluminescent AMH = a5+b5*Beckman AMH+c5*(Beckman AMH-1)+d5*(Beckman AMH-4);

if the Beckman AMH value is >13 ng/mL, then g1=1, g2=1, g3=1, and the formula is:

Kangrun chemiluminescent AMH = a5+b5*Beckman AMH+c5*(Beckman AMH-1)+d5*(Beckman AMH-4)+e5* (Beckman AMH-13),

wherein, a5 is any value selected from a range of -0.24101 to 0.26258; b5 is any value selected from a range of 0.72643 to 1.42037; c5 is any value selected from a range of -0.78107 to 0.04326; d5 is any value selected from a range of 0.29488 to 0.57880; e5 is any value selected from a range of -0.71455 to -0.47130;
preferably, the formula for converting Kangrun chemiluminescent AMH (X) to Beckman AMH (Y) is:

Beckman AMH = a6 + b6*Kangrun chemiluminescent AMH + c6*g1 (Kangrun chemiluminescent AMH-0.5) + d6*g2 (Kangrun chemiluminescent AMH-13);

in the formula, if the Kangrun chemiluminescent AMH value is $\leq 0.5$ ng/mL, then g1=0, g2=0, and the formula is:

$$\text{Beckman AMH} = a6 + b6 \cdot \text{Kangrun chemiluminescent AMH;}$$

if the Kangrun chemiluminescent AMH value is >0.5 and $\leq 13$ ng/mL, then g1=1, g2=0, and the formula is:

Beckman AMH = a6 + b6*Kangrun chemiluminescent AMH + c6*(Kangrun chemiluminescent AMH-0.5);

if the Kangrun chemiluminescent AMH value is >13 ng/mL, then g1=1, g2=1, and the formula is:

Beckman AMH = a6 + b6*Kangrun chemiluminescent AMH + c6*(Kangrun chemiluminescent AMH-0.5) + d6* (Kangrun chemiluminescent AMH-13);

wherein, a6 is any value selected from a range of -0.45082 to 0.54656; b6 is any value selected from a range of -0.35329 to 1.82169; c6 is any value selected from a range of -0.63742 to 1.56010; d6 is any value selected from a range of -0.52283 to -0.22757.

21. The system according to claim 17, wherein the sex hormone is anti-Müllerian hormone (AMH), and when the first platform is Kangrun chemiluminescent AMH detection platform and the second platform is Kangrun enzyme immunoassay (Anshlab) AMH detection platform, spline regression is used to convert the detection results of Kangrun chemiluminescent AMH detection platform and Kangrun enzyme immunoassay (Anshlab) AMH detection platform;

preferably, the formula for converting Kangrun chemiluminescent AMH (X) to Kangrun chemiluminescent AMH (Y) is:

Kangrun enzyme immunoassay (Anshlab) AMH = a7 + b7*Kangrun chemiluminescent AMH + c7*g1*(Kangrun chemiluminescent AMH-2) + d7*g2*(Kangrun chemiluminescent AMH-4) + e7*g3*(Kangrun chemiluminescent AMH-7);

wherein, a7 is any value selected from a range of -0.31000 to 0.20149, b7 is any value selected from a range of 0.73863 to 1.10064, c7 is any value selected from a range of -0.08941 to 0.51000, d7 is any value selected from a range of 0.08121 to 0.54582, and e7 is any value selected from a range of -0.70493 to -0.42738;
further,
if Kangrun chemiluminescent AMH is $\leq 2$ ng/mL, then g1 = 0, and the formula is:

Kangrun enzyme immunoassay (Anshlab) AMH = a7 + b7*Kangrun chemiluminescent AMH;

if Kangrun chemiluminescent AMH is >2 ng/mL and $\leq 4$ ng/mL, then g1=1, g2=0, and the formula is:

Kangrun enzyme immunoassay (Anshlab) AMH = a7 + b7*Kangrun chemiluminescent AMH + c7*g1 (Kangrun chemiluminescent AMH-2);

if Kangrun chemiluminescent AMH is >4 ng/mL and $\leq 7$ ng/mL, then g1=1, g2=1, and the formula is:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4);

if Kangrun chemiluminescent AMH is >7 ng/mL, then g1=1, g2=1, g3 = 1, and the formula is:

Kangrun enzyme immunoassay (Anshlab) AMH = a7 + b7*Kangrun chemiluminescent AMH + c7*g1 (Kangrun chemiluminescent AMH-2) + d7*g2 (Kangrun chemiluminescent AMH-4) + e7*g3 (Kangrun chemiluminescent AMH-7).

22. The system according to claim 17, wherein the sex hormone is estradiol $E_2$, and when the first platform is the Kangrun estradiol detection platform and the second platform is the Siemens estradiol detection platform, spline regression is used to convert the detection results of Kangrun estradiol and Siemens estradiol;

Siemens $E_2$ detection value = a8+b8*Kangrun $E_2$ detection value+c8*g1 (Kangrun $E_2$ detection value-660) + d8*g2 (Kangrun $E_2$ detection value-2030);

in the formula,

if Kangrun $E_2$ detection value is $\leq$ 660pmol/L, then g1=0, g2=0, and the formula is:

$$\text{Siemens } E_2 \text{ detection value} = a8+b8* \text{ Kangrun } E_2 \text{ detection value;}$$

if the Kangrun $E_2$ detection value is >660pmol/L and $\leq$2030pmol/L, then g1=1, g2=0, and the formula is:

Siemens $E_2$ detection value = a8+b8*Kangrun $E_2$ detection value + c8*(Kangrun $E_2$ detection value - 660);

if the Kangrun $E_2$ detection value is >2030pmol/L, then g1 = 1, g2 = 1, and the formula is:

Siemens $E_2$ detection value = a8+b8*Kangrun $E_2$ detection value+c8*(Kangrun $E_2$ detection value-660) + d8* (Kangrun $E_2$ detection value-2030);

wherein, a7 is any value selected from a range of -93.069 to -6.91207; b7 is any value selected from a range of 1.310892 to 1.569808; c7 is any value selected from a range of -0.8957 to -0.52322; d7 is any value selected from a range of -0.08505 to 0.085318.

Fig. 1

Fig. 2

Fig. 3

Fig. 4

Fig. 5

Fig. 6

Fig. 7

Fig. 8

Fig. 9

## INTERNATIONAL SEARCH REPORT

| International application No. |
|---|
| **PCT/CN2023/119663** |

| A. | CLASSIFICATION OF SUBJECT MATTER |
|---|---|

G16H 50/70(2018.01)i

According to International Patent Classification (IPC) or to both national classification and IPC

| B. | FIELDS SEARCHED |
|---|---|

Minimum documentation searched (classification system followed by classification symbols)

IPC: G16H

Documentation searched other than minimum documentation to the extent that such documents are included in the fields searched

Electronic data base consulted during the international search (name of data base and, where practicable, search terms used)

VEN, CNABS, CNTXT, WOTXT, USTXT, CNKI, IEEE: 检测, 激素, 转换, 回归, 拟合, 置信区间, 差异, 节点, detection, hormone, switch, regression, fit, confidence interval, differences, node

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 115831382 A (PEKING UNIVERSITY THIRD HOSPITAL (PEKING UNIVERSITY THIRD CLINICAL MEDICAL COLLEGE) et al.) 21 March 2023 (2023-03-21) <br> claims 1-11, and description, paragraphs 0098-0300 | 1-22 |
| A | CN 110491505 A (PEKING UNIVERSITY THIRD HOSPITAL (PEKING UNIVERSITY THIRD CLINICAL MEDICAL COLLEGE)) 22 November 2019 (2019-11-22) <br> description, paragraphs [0120]-[0172] | 1-22 |
| A | CN 115019932 A (PEKING UNIVERSITY THIRD HOSPITAL (PEKING UNIVERSITY THIRD CLINICAL MEDICAL COLLEGE)) 06 September 2022 (2022-09-06) <br> entire document | 1-22 |
| A | CN 114913972 A (PEKING UNIVERSITY THIRD HOSPITAL (PEKING UNIVERSITY THIRD CLINICAL MEDICAL COLLEGE) et al.) 16 August 2022 (2022-08-16) <br> entire document | 1-22 |
| A | US 2021158899 A1 (JAPAN SCIENCE AND TECHNOLOGY AGENCY) 27 May 2021 (2021-05-27) <br> entire document | 1-22 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **15 November 2023** | **23 November 2023** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/119663**

| Patent document cited in search report | | | Publication date (day/month/year) | Patent family member(s) | | | Publication date (day/month/year) |
|---|---|---|---|---|---|---|---|
| CN | 115831382 | A | 21 March 2023 | None | | | |
| CN | 110491505 | A | 22 November 2019 | WO | 2021031605 | A1 | 25 February 2021 |
| CN | 115019932 | A | 06 September 2022 | None | | | |
| CN | 114913972 | A | 16 August 2022 | None | | | |
| US | 2021158899 | A1 | 27 May 2021 | WO | 2018207925 | A1 | 15 November 2018 |
| | | | | JPWO | 2018207925 | A1 | 19 March 2020 |
| | | | | JP | 7124265 | B2 | 24 August 2022 |

Form PCT/ISA/210 (patent family annex) (July 2022)